# EUROPÄISCHE PATENTSCHRIFT

(11) **EP 1 759 013 B1**
(45) Veröffentlichungstag und Bekanntmachung des Hinweises auf die Patenterteilung: **19.05.2010**
(21) Anmeldenummer: 05747772.1
(22) Anmeldetag: 11.05.2005
(51) Int. Cl.: C12Q 1/68

(54) **IDENTIFIZIERUNG VON OBERFLÄCHEN-ASSOZIIERTEN ANTIGENEN FÜR DIE TUMORDIAGNOSE UND -THERAPIE**
IDENTIFICATION OF SURFACE-ASSOCIATED ANTIGENS FOR TUMOR DIAGNOSIS AND THERAPY
IDENTIFICATION D'ANTIGENES DE SURFACE POUR LE DIAGNOSTIC ET LA THERAPIE DE TUMEURS

(30) Priorität: 11.05.2004 DE 102004023187
(43) Veröffentlichungstag der Anmeldung: 07.03.2007
(73) Patentinhaber: Ganymed Pharmaceuticals AG, 55131 Mainz (DE)
(72) Erfinder: TÜRECI, Özlem, 55116 Mainz (DE); SAHIN, Ugur, 55116 Mainz (DE); SCHNEIDER, Sandra, 76297 Stutensee (DE); HELFTENBEIN, Gerd, 35329 Gemünden (DE); SCHLÜTER, Volker, 82061 Neuried (DE); USENER, Dirk, 65193 Wiesbaden (DE); THIEL, Philippe, 82152 Planegg (DE); KOSLOWSKI, Michael, 55116 Mainz (DE)
(74) Vertreter: Schnappauf, Georg
(86) Internationale Anmeldenummer: PCT/EP2005/005104
(87) Internationale Veröffentlichungsnummer: WO 2005/110338

(56) Entgegenhaltungen:
- WO-A-03/000928
- WO-A-03/076631
- WO-A-2004/047863
- DATABASE EMBL [Online] 28. Mai 2002 (2002-05-28), "AGENCOURT_7926639 NIH_MGC_72 Homo sapiens cDNA clone IMAGE:6160718 5', mRNA sequence." XP002358303 gefunden im EBI accession no. EM_EST:BQ427878 Database accession no. BQ427878
- DATABASE EMBL [Online] 15. April 2003 (2003-04-15), "Sequence 695 from Patent EP1293569." XP002358304 gefunden im EBI accession no. EM_PAT:AX714011 Database accession no. AX714011 & EP 1 293 569 A (RESEARCH ASSOCIATION FORBIOTECHNOLOGY) 19. März 2003 (2003-03-19)
- DATABASE EMBL [Online] 31. Oktober 2001 (2001-10-31), "Homo sapiens cDNA FLJ31461 fis, clone NT2NE2001247." XP002358305 gefunden im EBI accession no. EM_HUM:AK056023 Database accession no. AK056023
- DATABASE UniProt [Online] 1. Dezember 2001 (2001-12-01), "Hypothetical protein FLJ31461." XP002358306 gefunden im EBI accession no. UNIPROT:Q96N35 Database accession no. Q96N35
- SCHEURLE DANIELA ET AL: "Cancer gene discovery using Digital Differential Display" CANCER RESEARCH, AMERICAN ASSOCIATION FOR CANCER RESEARCH, BALTIMORE, MD, US, Bd. 60, Nr. 15, 1. August 2000 (2000-08-01), Seiten 4037-4043, XP002256957 ISSN: 0008-5472

## Beschreibung

Trotz interdisziplinärer Ansätze und Ausreizung klassischer Therapiemodalitäten gehören Krebserkrankungen weiterhin zu den führenden Todesursachen. Neuere therapeutische Konzepte zielen darauf ab, das patienteneigene Immunsystem durch Einsatz von rekombinanten Tumorvakzinen und anderen spezifischen Maßnahmen wie Antikörpertherapie in das therapeutische Gesamtkonzept mit einzubeziehen. Voraussetzung für den Erfolg einer solchen Strategie ist die Erkennung von Tumor-spezifischen oder Tumor-assoziierten Antigenen bzw. Epitopen durch das Immunsystem des Patienten, dessen Effektorfunktionen verstärkt werden sollen. Tumorzellen unterscheiden sich biologisch wesentlich von ihren nichtmalignen Ursprungszellen. Diese Differenzen sind durch während der Tumorentwicklung erworbene genetische Veränderungen bedingt und führen u.a. auch zur der Bildung qualitativ oder quantitativ veränderter molekularer Strukturen in den Krebszellen. Werden solche Tumor-assoziierten Strukturen vom spezifischen Immunsystem des tumortragenden Wirtes erkannt, spricht man von Tumor-assoziierten Antigenen.

An der spezifischen Erkennung von Tumor-assoziierten Antigenen sind zelluläre und humorale Mechanismen beteiligt, die zwei miteinander funktionell vernetzte Einheiten darstellen: CD4⁺ und CD8⁺ T-Lymphozyten erkennen prozessierte Antigene, die auf den Molekülen der MHC- (Major Histocompatibility complex = Histokompatibilitäts-Antigene) Klassen II bzw. I präsentiert werden, während B-Lymphozyten zirkulierende Antikörpermoleküle produzieren, die direkt an unprozessierte Antigene binden.

Die potentielle klinisch-therapeutische Bedeutung von Tumor-assoziierten Antigenen ergibt sich aus der Tatsache, dass die Erkennung von Antigenen auf neoplastischen Zellen durch das Immunsystem zur Initiierung von cytotoxischen Effektormechanismen führt und bei-Vorhandensein von T-Helferzellen die Elimination der Krebszellen bewirken kann (Pardoll, Nat. Med. 4:525-31, 1998). Entsprechend ist es eine zentrale Zielsetzung der Tumorimmunologie, diese Strukturen molekular zu definieren. Die molekulare Natur dieser Antigene blieb lange enigmatisch. Erst als entsprechende Klonierungstechniken entwickelt wurden, gelang es, durch Analyse der Zielstrukturen von cytotoxischen T-Lymphozyten (CTL) (van der Bruggen et al., Science 254:1643-7, 1991) bzw. mit zirkulierenden Autoantikörpern (Sahin et al., Curr. Opin. Immunol. 9:709-16, 1997) als Sonden cDNA-Expressionsbanken von Tumoren systematisch auf Tumor-assoziierte Antigene zu analysieren. Hierzu wurden cDNA-Expressionsbanken aus frischem Tumorgewebe hergestellt und in geeigneten Systemen als Proteine rekombinant exprimiert. Aus Patienten isolierte Immuneffektoren, nämlich CTL-Klone mit Tumor-spezifischem Lysemuster, oder zirkulierende Autoantikörper wurden genutzt, um die respektiven Antigene zu klonieren.

Durch diese Ansätze sind in den letzten Jahren eine Vielzahl von Antigenen in verschiedenen Neoplasien definiert worden. Von großem Interesse ist dabei die Klasse der Cancer/Testis-Antigene (CTA). CTA und sie kodierende Gene (Cancer/Testis-Gene oder CTG) sind durch ihr charakteristisches Expressionsmuster definiert (Türeci et al, Mol. Med. Today 3:342-9, 1997). Sie finden sich nicht in Normalgeweben bis auf Testis bzw. Keimzellen, werden jedoch in einer Reihe von humanen Malignomen exprimiert und zwar nicht tumortypspezifisch, sondern mit unterschiedlicher Häufigkeit in Tumorentitäten ganz unterschiedlicher Herkunft (Chen & Old, Cancer J. Sci. Am. 5:16-7, 1999). Antikörper gegen CTA sind in gesunden Personen nicht nachweisbar, wohl aber in Tumorpatienten. Insbesondere aufgrund ihrer Gewebeverteilung ist diese Antigenklasse von besonderem Wert für immuntherapeutische Vorhaben und wird in derzeit laufenden klinischen Patientenstudien getestet (Marchand et al., Int. J. Cancer 80:219-30, 1999; Knuth et al., Cancer Chemother. Pharmacol. 46: S46-S1, 2000).

Allerdings nutzen die oben dargestellten klassischen Verfahren zur Antigenidentifizierung Immuneffektoren (zirkulierende Autoantikörper oder CTL-Klone) aus Patienten mit in der Regel bereits fortgeschrittenem Krebs als Sonden. Aus einer Reihe von Daten geht hervor, dass Tumore z.B. zur Tolerisierung und Anergisierung von T-Zellen führen können und gerade im Verlauf der Erkrankung diejenigen Spezifitäten aus dem Immuneffektorenrepertoire verloren gehen, die eine effektive Immunerkennung bewirken könnten. Aus laufenden Patientenstudien hat sich noch kein gesicherter Beweis für eine tatsächliche, Wirkung der bisher entdeckten und genutzten Tumor-assoziierten Antigene ergeben. Entsprechend kann nicht ausgeschlossen werden, dass spontane Immunantworten evozierende Proteine die falschen Zielstrukturen sind.

DATABASE EMBL [Online] 28. Mai 2002, Database accession no. BQ427878, DATABASE EMBL [Online] 15. April 2003, Database accession no. AX714011, DATABASE EMBL [Online] 31. Oktober 2001, Database accession no. AK056023 und DATABASE UniProt [Online] 1. Dezember 2001, Database accession no. Q96N35 sind Datenbankeinträge, die Nukleinsäuresequenzen bzw. eine Aminosäuresequenz offenbaren. Über die Funktion oder eine mögliche Verwendung dieser Sequenzen wird nichts ausgesagt. WO 03/076631 beschreibt differentiell in Tumoren exprimierte Genprodukte und deren Verwendung. WO 03/000928 beschreibt Verfahren zur Identifizierung von Molekülen mit einem unterschiedlichen Grad an Expression auf der Oberfläche von Krebszellen im Vergleich zu nicht-malignen Zellen.

Es war die Aufgabe der vorliegenden Erfindung Zielstrukturen für eine Diagnose und Therapie von Krebserkrankungen bereitzustellen.

Diese Aufgabe wird erfindungsgemäß durch den Gegenstand der Patentansprüche gelöst.

Erfindungsgemäß wurde eine Strategie für eine Identifizierung und Bereitstellung Tumor-assoziierter Antigene und der dafür kodierenden Nukleinsäuren verfolgt. Diese Strategie beruht auf der Auswertung humaner Protein- und Nukleinsäuredatenbanken im Hinblick auf potenzielle, krebsspezifische Antigene, die auf der Zelloberfläche zugänglich sind. Die Definition der dafür notwendigen Filterkriterien zusammen mit einer Hochdurchsatz-Methodik zur Analyse möglichst aller Gene bildet den zentralen Bestandteil der Erfindung. Durch Datamining wird zunächst eine möglichst komplette Liste aller bekannter Gene aufgestellt, die dem Grundprinzip Gen zu mRNA zu Protein folgend auf das Vorhandensein einer oder mehrerer Transmembrandomänen hin untersucht werden. Hieran schließen sich eine Homologiesuche, eine Einteilung der Treffer in gewebsspezifische Gruppen (u.a. Tumorgewebe) und eine Überprüfung der realen Existenz der mRNA an. Schließlich werden die so identifizierten Proteine z.B. durch Expressionsanalysen und proteinchemische Verfahren auf ihre aberrante Aktivierung in Tumoren evaluiert.

Datamining ist ein bekanntes Verfahren zur Identifizierung von Tumor-assoziierten Genen. Bei den herkömmlichen Strategien werden allerdings in der Regel Transkriptome von Normalgewebebanken elektronisch von Tumorgewebsbanken unter der Annahme subtrahiert, dass die verbleibenden Gene Tumor-spezifisch sind (Schmitt et al., Nucleic Acids Res. 27:4251-60, 1999; Vasmatzis et al., Proc. Natl. Acad. Sci. USA. 95:300-4, 1998; Scheurle et al., Cancer Res. 60:4037-43, 2000).

Das erfindungsgemäße Konzept beruht jedoch darauf, Datamining zur elektronischen Extraktion aller Gene, die für auf der Zelloberfläche zugängliche, krebsspezifische Antigene kodieren, zu nutzen und diese sodann auf ektope Expression in Tumoren zu evaluieren.

Die Strategie zur Identifizierung von differentiell in Tumoren exprimierten Genen kombiniert Datamining von öffentlichen Sequenzbanken ("*in silico")* mit darauffolgenden laborexperimentellen ("wet bench") Untersuchungen.

Eine kombinierte Strategie basierend auf unterschiedlichen bioinformatischen Skripten ermöglichte die Identifizierung von Genen, die für auf der Zelloberfläche zugängliche, krebsspezifische Antigene kodieren. Die Identifizierung dieser Tumor-assoziierten Gene und der dadurch kodierten Genprodukte erfolgte unabhängig von einer immunogenen Wirkung.

Die Eigenschaft der identifizierten Tumor-assoziierten Antigene, dass sie auf oder an der Zelloberfläche lokalisiert sind, qualifiziert sie als geeignete Ziele oder Mittel für die Therapie und Diagnose. Besonders geeignet hierfür ist ein Teil der identifizierten Tumor-assoziierten Antigene, der dem nicht-Transmembrananteil, insbesondere dem extrazellulären Anteil der Antigene entspricht oder davon umfasst wird. Somit ist ein Teil der identifizierten Tumor-assoziierten Antigene, der dem nicht-Transmembrananteil der Antigene entspricht oder davon umfasst ist, oder ein entsprechender Teil der für die identifizierten Antigene kodierenden Nukleinsäuren für eine Therapie oder Diagnose bevorzugt. Ähnlich ist die Verwendung von Antikörpern bevorzugt, die gegen einen Teil der identifizierten Tumor-assoziierten Antigene gerichtet sind, der dem nicht-Transmembrananteil der Antigene entspricht oder davon umfasst ist.

In einem Aspekt betrifft die Erfindung eine pharmazeutische Zusammensetzung, umfassend einen oder mehrere Bestandteile, die aus der Gruppe ausgewählt sind, bestehend aus:
(i) einem Antikörper, der an ein Tumor-assozüertes Antigen bindet,
(ii) einem Tumor-assoziierten Antigen oder einem Teil davon, der mindestens 6 aufeinanderfolgende Aminosäuren umfasst,
(iii) einer Nukleinsäure, die für ein Tumor-assoziiertes Antigen oder einen Teil davon, der mindestens 6 aufeinanderfolgende Aminosäuren umfasst, kodiert,
(iv) einer Wirtszelle, die ein Tumor-assoziiertes Antigen oder einen Teil davon, der mindestens 6 aufeinanderfolgende Aminosäuren umfasst, exprimiert, und
(v) isolierten Komplexen zwischen einem Tumor-assoziierten Antigen oder einem Teil davon und einem HLA-Molekül,
wobei das Tumor-assoziierte Antigen
(a) die Aminosäuresequenz gemäß SEQ ID NO: 10 umfasst, oder
(b) eine Sequenz aufweist, die von einer Nukleinsäure kodiert wird, die mindestens 90% Identität zu der Nukleinsäure gemäß SEQ ID NO: 9 aufweist,
zur Behandlung von und/oder Vakzinierung gegen Krebs, der sich durch die Expression oder abnormale Expression des Tumor-assoziierten Antigens auszeichnet, wobei der Krebs ausgewählt ist aus der Gruppe bestehend aus Brusttumor, Lungentumor, Ösophagustumor, Kolontumor, Magentumor, Pankreastumor, Lebertumor, Ovarialtumor, Prostatatumor, HNO-Tumor, Nierentumor, Zervixtumor, Hauttumor und Uterustumor.

In einer bevorzugten Ausführungsform bindet der Antikörper selektiv an das Tumor-assoziierte Antigen, weist tumorhemmende Aktivität auf und ist selektiv für Zellen, die eine Expression oder abnormale Expression des Tumor-assoziierten Antigens aufweisen.

In einer bevorzugten Ausführungsform erhöhen die Bestandteile unter (ii), (iii), (iv) und (v) selektiv die Menge an Komplexen zwischen einem HLA-Molekül und dem Tumor-assoziierten Antigen oder einem Teil davon.

In einer bevorzugten Ausführungsform liegt die Nukleinsäure unter (iii) in einem Expressionsvektor vor.

In einer bevorzugten Ausführungsform sekretiert die Wirtszelle das Tumor-assoziierte Antigen oder den Teil davon. In einer weiteren bevorzugten Ausführungsform exprimiert die Wirtszelle zusätzlich ein HLA-Molekül, das an das Tumor-assoziierte Antigen oder den Teil davon bindet, wobei die Wirtszelle vorzugsweise das HLA-Molekül und/oder das Tumor-assoziierte Antigen oder den Teil davon rekombinant exprimiert oder das HLA-Molekül endogen exprimiert. Vorzugsweise ist die Wirtszelle nicht-proliferativ. Die Wirtszelle ist vorzugsweise eine Antigen-präsentierende Zelle.

In einer bevorzugten Ausführungsform ist der Antikörper ein monoklonaler, chimärer oder humanisierter Antikörper oder ein Fragment eines Antikörpers. Vorzugsweise ist der Antikörper mit einem therapeutischen oder diagnostischen Mittel gekoppelt.

In bestimmten Ausführungsformen bindet ein durch eine erfindungsgemäße pharmazeutische Zusammensetzung entweder direkt oder durch die Expression einer Nukleinsäure bereitgestelltes Tumor-assoziiertes Antigen oder ein Teil davon an MHC-Moleküle auf der Oberfläche von Zellen, wobei die Bindung vorzugsweise eine cytolytische Reaktion hervorruft und/oder eine Zytokinausschüttung induziert.

Eine erfindungsgemäße pharmazeutische Zusammensetzung kann einen pharmazeutisch verträglichen Träger und/oder ein Adjuvans umfassen. Das Adjuvans kann aus Saponin, GM-CSF, CpG-Oligonukleotiden, RNA, einem Zytokin oder einem Chemokin ausgewählt sein.

In einem zweiten Aspekt betrifft die Erfindung ein Verfahren zur Diagnose einer Krebserkrankung, die sich durch die Expression oder abnormale Expression eines Tumor-assoziierten Antigens auszeichnet, umfassend den Nachweis:
(i) einer Nukleinsäure, die für das Tumor-assoziierte Antigen kodiert, und/oder
(ii) des Tumor-assoziierten Antigens
in einer aus einem Patienten isolierten biologischen Probe,
wobei das Tumor-assoziierte Antigen
(a) die Aminosäuresequenz gemäß SEQ ID NO: 10 umfasst, oder
(b) eine Sequenz aufweist, die von einer Nukleinsäure kodiert wird, die mindestens 90% Identität zu der Nukleinsäure gemäß SEQ ID NO: 9 aufweist,
wobei die Krebserkrankung ausgewählt ist aus der Gruppe bestehend aus Brusttumor, Lungentumor, Ösophagustumor, Kolontumor, Magentumor, Pankreastumor, Lebertumor, Ovarialtumor, Prostatatumor, HNO-Tumor, Nierentumor, Zervixtumor, Hauttumor und Uterustumor.

In bestimmten Ausführungsformen umfasst der Nachweis (i) die Kontaktierung der biologischen Probe mit einem Mittel, das spezifisch an die Nukleinsäure, die für das Tumor-assoziierte Antigen kodiert, oder an das Tumor-assoziierte Antigen bindet und (ii) den Nachweis der Komplexbildung zwischen dem Mittel und der Nukleinsäure oder dem Tumor-assoziierten Antigen. In einer weiteren Ausführungsform wird die isolierte biologische Probe aus dem Patienten mit einer vergleichbaren normalen biologischen Probe verglichen.

In einem dritten Aspekt betrifft die Erfindung ein Verfahren zur Bestimmung der Regression, des Verlaufs oder des Ausbruchs einer Krebserkrankung, die sich durch die Expression oder abnormale Expression eines Tumor-assoziierten Antigens auszeichnet, umfassend die Überwachung einer Probe aus einem Patienten, der die Krebserkrankung aufweist oder in Verdacht steht, an der Krebserkrankung zu erkranken, in Bezug auf einen oder mehrere Parameter, ausgewählt aus der Gruppe, bestehend aus:
(i) der Menge der Nukleinsäure, die für das Tumor-assoziierte Antigen kodiert, und
(ii) der Menge des Tumor-assoziierten Antigens,
wobei das Tumor-assoziierte Antigen
(a) die Aminosäuresequenz gemäß SEQ ID NO: 10 umfasst, oder
(b) eine Sequenz aufweist, die von einer Nukleinsäure kodiert wird, die mindestens 90% Identität zu der Nukleinsäure gemäß SEQ ID NO: 9 aufweist,
wobei die Krebserkrankung ausgewählt ist aus der Gruppe bestehend aus Brusttumor, Lungentumor, Ösophagustumor, Kolontumor, Magentumor, Pankreastumor, Lebertumor, Ovarialtumor, Prostatatumor, HNO-Tumor, Nierentumor, Zervixtumor, Hauttumor und Uterustumor.

In einer bevorzugten Ausführungsform des Verfahrens gemäß dem dritten Aspekt umfasst das Verfahren die Bestimmung des oder der Parameter zu einem ersten Zeitpunkt in einer ersten Probe und zu einem zweiten Zeitpunkt in einer weiteren Probe und durch einen Vergleich der beiden Proben wird der Verlauf der Krebserkrankung ermittelt.

In einer bevorzugten Ausführungsform des Verfahrens gemäß dem zweiten und dritten Aspekt erfolgt der Nachweis oder die Überwachung gemäß (i) durch selektive Amplifikation der Nukleinsäure oder eines Teils davon oder mit einer Polynukleotid-Sonde, die spezifisch mit der Nukleinsäure hybridisiert. In einer Ausführungsform umfasst die Polynukleotid-Sonde eine Sequenz von 6-50, insbesondere 10-30, 15-30 oder 20-30 zusammenhängenden Nukleotiden aus der Nukleinsäure.

In einer bevorzugten Ausführungsform des Verfahrens gemäß dem zweiten und dritten Aspekt erfolgt der Nachweis oder die Überwachung gemäß (ii) mit einem Antikörper, der spezifisch an das Tumor-assoziierte Antigen bindet.

Die für einen Nachweis oder für eine Überwachung verwendete Polynukleotid-Sonde oder der Antikörper ist vorzugsweise nachweisbar markiert. In bestimmten Ausführungsformen ist der nachweisbare Marker ein radioaktiver Marker oder ein Enzymmarker.

In einem vierten Aspekt betrifft die Erfindung eine Verwendung eines Antikörpers, der an ein Tumor-assoziiertes Antigen bindet und mit einem therapeutischen oder diagnostischen Mittel gekoppelt ist, zur Herstellung einer pharmazeutischen Zusammensetzung zur Behandlung, Diagnose oder Überwachung einer Krebserkrankung, die sich durch die Expression oder abnormale Expression des Tumor-assoziierten Antigens auszeichnet,
wobei das Tumor-assoziierte Antigen
(a) die Aminosäuresequenz gemäß SEQ ID NO: 10 umfasst, oder
(b) eine Sequenz aufweist, die von einer Nukleinsäure kodiert wird, die mindestens 90% Identität zu der Nukleinsäure gemäß SEQ ID NO: 9 aufweist,
wobei die Krebserkrankung ausgewählt ist aus der Gruppe bestehend aus Brusttumor, Lungentumor, Ösophagustumor, Kolontumor, Magentumor, Pankreastumor, Lebertumor, Ovarialtumor, Prostatatumor, HNO-Tumor, Nierentumor, Zervixtumor, Hauttumor und Uterustumor.

In einer bevorzugten Ausführungsform des erfindungsgemäßen Verfahrens oder der erfindungsgemäßen Verwendung ist der Antikörper ein monoklonaler, chimärer oder humanisierten Antikörper oder ein Fragment eines Antikörpers.

Oligonukleotide, die mit einer erfindungsgemäß identifizierten Nukleinsäure hybridisieren, können als genetische Sonden verwendet werden. Die Hybridisierung kann unter niedrig-, besser unter mittel- und am besten unter hoch-stringenten Bedingungen erfolgen. Der Begriff "stringente Bedingungen" betrifft erfindungsgemäß Bedingungen, die eine spezifische Hybridisierung zwischen Polynukleotiden erlauben.

### Detaillierte Beschreibung der Erfindung

Therapeutische Konzepte betrachten Tumor-assoziierte Antigene als Markierungen, die Effektormechanismen mit zellschädigendem Potential selektiv zu Tumorzellen rekrutieren. Hierbei ist die Funktion des Zielmoleküls selbst und seine Rolle bei der Tumorentstehung vollkommen unerheblich.

Mit "Derivat" einer Nukleinsäure ist erfindungsgemäß gemeint, dass einzelne oder multiple Nukleotidsubstitutionen, -deletionen und/oder -additionen in der Nukleinsäure vorliegen. Weiterhin umfasst der Begriff "Derivat" auch eine chemische Derivatisierung einer Nukleinsäure an einer Base, einem Zucker oder Phosphat eines Nukleotids. Der Begriff "Derivat" umfasst auch Nukleinsäuren, die nicht in der Natur vorkommende Nukleotide und Nukleotidanaloga enthalten.

Eine Nukleinsäure ist erfindungsgemäß vorzugsweise Desoxyribonukleinsäure (DNA) oder Ribonukleinsäure (RNA). Nukleinsäuren umfassen erfindungsgemäß genomische DNA, cDNA, mRNA, rekombinant hergestellte und chemisch synthetisierte Moleküle. Eine Nukleinsäure kann erfindungsgemäß als einzelsträngiges oder doppelsträngiges und lineares oder kovalent kreisförmig geschlossenes Molekül vorliegen.

Die erfindungsgemäß beschriebenen Nukleinsäuren sind vorzugsweise isoliert. Der Begriff "isolierte Nukleinsäure" bedeutet erfindungsgemäß, dass die Nukleinsäure (i) *in vitro* amplifiziert wurde, zum Beispiel durch Polymerase-Kettenreaktion (PCR), (ii) rekombinant durch Klonierung produziert wurde, (iii) gereinigt wurde, zum Beispiel durch Spaltung und gelelektrophoretische Auftrennung, oder (iv) synthetisiert wurde, zum Beispiel durch chemische Synthese. Eine isolierte Nukleinsäure ist eine Nukleinsäure, die für eine Manipulierung durch rekombinante DNA-Techniken zur Verfügung steht.

Eine Nukleinsäure ist dann zu einer anderen Nukleinsäure "komplementär", wenn die beiden Sequenzen miteinander hybridisieren und ein stabiles Duplexmolekül eingehen können, wobei die Hybridisierung vorzugsweise unter Bedingungen erfolgt, die eine spezifische Hybridisierung zwischen Polynukleotiden erlauben (stringente Bedingungen). Stringente Bedingungen sind beispielsweise in Molecular Cloning: A Laboratory Manual, J. Sambrook et al., Hrsg., 2. Auflage, Cold Spring Harbor Laboratory Press, Cold Spring Harbor, New York, 1989, oder Current Protocols in Molecular Biology, F.M. Ausubel et al., Hrsg., John Wiley & Sons, Inc., New York beschrieben und betreffen beispielsweise die Hybridisierung bei 65°C in Hybridisierungspuffer (3,5 x SSC, 0,02% Ficoll, 0,02% Polyvinylpyrrolidon, 0,02% Rinderserumalbumin, 2,5 mM NaH₂PO₄ (pH 7), 0,5% SDS, 2 mM EDTA). SSC ist eine Lösung mit jeweils 0,15 M Natriumchlorid und Natriumcitrat, pH 7. Nach der Hybridisierung wird die Membran, auf die die DNA übertragen wurde, beispielsweise in 2 x SSC bei Raumtemperatur und sodann in 0,1 - 0,5 x SSC / 0,1 x SDS bei Temperaturen bis 68°C gewaschen.

Komplementäre Nukleinsäuren weisen erfindungsgemäß mindestens 40%, insbesondere mindestens 50%, mindestens 60%, mindestens 70%, mindestens 80%, mindestens 90% und vorzugsweise mindestens 95%, mindestens 98% oder mindestens 99% Identität der Nukleotide auf.

Nukleinsäuren, die für Tumor-assoziierte Antigene kodieren, können erfindungsgemäß alleine oder in Kombination mit anderen Nukleinsäuren, insbesondere heterologen Nukleinsäuren, vorliegen. In bevorzugten Ausführungsformen liegt eine Nukleinsäure funktionell in Verbindung mit Expressionskontrollsequenzen oder regulatorischen Sequenzen vor, die in Bezug zu der Nukleinsäure homolog oder heterolog sein können. Eine kodierende Sequenz und eine regulatorische Sequenz sind dann "funktionell" miteinander verbunden, falls sie derart kovalent miteinander verknüpft sind, dass die Expression oder Transkription der kodierenden Sequenz unter der Kontrolle oder unter dem Einfluss der regulatorischen Sequenz steht. Falls die kodierende Sequenz in ein funktionelles Protein translatiert werden soll, führt bei einer funktionellen Verbindung einer regulatorischen Sequenz mit der kodierenden Sequenz eine Induktion der regulatorischen Sequenz zu einer Transkription der kodierenden Sequenz, ohne dass es zu einer Leserasterverschiebung in der kodierenden Sequenz oder zu einem Unvermögen der kodierenden Sequenz kommt, in das gewünschte Protein oder Peptid translatiert zu werden.

Der Begriff "Expressionskontrollsequenz" oder "regulatorische Sequenz" umfasst erfindungsgemäß Promotoren, Enhancer und andere Kontrollelemente, die die Expression eines Gens steuern. In bestimmten erfindungsgemäßen Ausführungsformen sind die Expressionskontrollsequenzen regulierbar. Die genaue Struktur von regulatorischen Sequenzen kann speziesabhängig oder zelltypusabhängig variieren, umfasst jedoch im allgemeinen 5'-nicht-transkribierte und 5'-nicht-translatierte Sequenzen, die an der Initiation der Transkription bzw. Translation beteiligt sind wie TATA-Box, Capping-Sequenz, CAAT-Sequenz und ähnliches. Insbesondere umfassen 5'-nicht-transkribierte Regulationssequenzen eine Promotorregion, die eine Promotorsequenz für eine transkriptionelle Kontrolle des funktionell verbundenen Gens einschließt. Regulatorische Sequenzen können auch EnhancerSequenzen oder stromaufwärts gelegene Aktivatorsequenzen umfassen.

Zum einen können also die hier dargestellten Tumor-assoziierten Antigene mit beliebigen Expressionskontrollsequenzen und Promotoren kombiniert werden. Zum anderen aber können erfindungsgemäß die Promotoren der hier dargestellten Tumor-assoziierten Genprodukte mit beliebigen anderen Genen kombiniert werden. Dies erlaubt, die selektive Aktivität dieser Promotoren zu nutzen.

Des Weiteren kann eine Nukleinsäure erfindungsgemäß in Verbindung mit einer anderen Nukleinsäure vorliegen, die für ein Polypeptid kodiert, das eine Sekretion des durch die Nukleinsäure kodierten Proteins oder Polypeptids aus einer Wirtszelle steuert. Auch kann eine Nukleinsäure erfindungsgemäß in Verbindung mit einer anderen Nukleinsäure vorliegen, die für ein Polypeptid kodiert, das eine Verankerung des kodierten Proteins oder Polypeptids auf der Zellmembran der Wirtszelle oder seine Kompartimentalisierung in bestimmte Organellen dieser Zelle herbeiführt.

In einer bevorzugten Ausführungsform ist ein rekombinantes DNA-Molekül erfindungsgemäß ein Vektor, gegebenenfalls mit einem Promotor, der die Expression einer Nukleinsäure, z.B. einer Nukleinsäure, die für ein erfindungsgemäßes Tumor-assozüertes Antigen kodiert, steuert. Der Begriff "Vektor" wird dabei in seiner allgemeinsten Bedeutung verwendet und umfasst jegliche intermediären Vehikel für eine Nukleinsäure, die es z.B. ermöglichen, die Nukleinsäure in prokaryontische und/oder in eukaryontische Zellen einzubringen und gegebenenfalls in ein Genom zu integrieren. Solche Vektoren werden vorzugsweise in der Zelle repliziert und/oder exprimiert. Ein intermediäres Vehikel kann z.B. für den Gebrauch bei der Elektroporation, beim Mikroprojektilbeschuss, bei der liposomalen Verabreichung, beim Transfer mit Hilfe von Agrobakterien oder bei der Insertion über DNA- oder RNA-Viren angepasst sein. Vektoren umfassen Plasmide, Phagemide, Bacteriophage oder Virusgenome.

Die Nukleinsäuren, die für ein erfindungsgemäß identifiziertes Tumor-assoziiertes Antigen kodieren, können für eine Transfektion von Wirtszellen eingesetzt werden. Mit Nukleinsäuren ist dabei sowohl.rekombinante DNA wie auch RNA gemeint. Rekombinante RNA kann durch *in vitro*-Transkription von einer DNA-Matritze hergestellt werden. Sie kann des weiteren vor Applikation durch stabilisierende Sequenzen, Capping und Poly-Adenylierung modifiziert werden.

Der Begriff "Wirtszelle" betrifft erfindungsgemäß jede Zelle, die mit einer exogenen Nukleinsäure transformierbar oder transfizierbar ist. Der Begriff "Wirtszellen" umfasst erfindungsgemäß prokaryontische (z.B. *E. coli*) oder eukaryontische (z.B. dendritische Zellen, B-Zellen, CHO-Zellen, COS-Zellen, K562-Zellen, Hefezellen und Insektenzellen). Besonders bevorzugt sind Säugerzellen wie Zellen aus Mensch, Maus, Hamster, Schwein, Ziege und Primaten. Die Zellen können aus einer Vielzahl von Gewebetypen abgeleitet sein und umfassen primäre Zellen und Zelllinien. Spezifische Beispiele umfassen Keratinozyten, periphere Blutleukozyten, Stammzellen des Knochenmarks und embryonale Stammzellen. In weiteren Ausführungsformen ist die Wirtszelle eine Antigen-präsentierende Zelle, insbesondere eine dendritische Zelle, ein Monozyt oder Makrophage. Eine Nukleinsäure kann in der Wirtszelle in einer einzigen oder in mehreren Kopien vorliegen und wird in einer Ausführungsform in der Wirtszelle exprimiert.

Der Begriff "Expression" wird erfindungsgemäß in seiner allgemeinsten Bedeutung verwendet und umfasst die Produktion von RNA oder von RNA und Protein. Er umfasst auch eine teilweise Expression von Nukleinsäuren. Des Weiteren kann die Expression transient oder stabil erfolgen. Bevorzugte Expressionssysteme in Säugerzellen umfassen pcDNA3.1 und pRc/CMV (Invitrogen, Carlsbad, CA), die einen selektierbaren Marker enthalten wie ein Gen, das eine Resistenz gegenüber. G418 verleiht (und somit eine Selektion stabil transfizierter Zelllinien ermöglicht), und die Enhancer-Promotor-Sequenzen von Cytomegalovirus (CMV).

In den Fällen der Erfindung, in denen ein HLA-Molekül ein Tumor-assoziiertes Antigen oder einen Teil davon präsentiert, kann ein Expressionsvektor auch eine Nukleinsäuresequenz umfassen, die für das HLA-Molekül kodiert. Die Nukleinsäuresequenz, die für das HLA-Molekül kodiert, kann auf demselben Expressionsvektor wie die Nukleinsäure, die für das Tumor-assoziierte Antigen oder den Teil davon kodiert, vorliegen oder beide Nukleinsäuren können auf verschiedenen Expressionsvektoren vorliegen. Im letzteren Fall können die beiden Expressionsvektoren in eine Zelle cotransfiziert werden. Falls eine Wirtszelle weder das Tumor-assoziierte Antigen oder den Teil davon noch das HLA-Molekül exprimiert, werden beide dafür kodierenden Nukleinsäuren entweder auf demselben Expressionsvektor oder auf verschiedenen Expressionsvektoren in die Zelle transfiziert. Falls die Zelle bereits das HLA-Molekül exprimiert, kann nur die Nukleinsäuresequenz, die für das Tumor-assoziierte Antigen oder den Teil davon kodiert, in die Zelle transfiziert werden.

Kits zur Amplifikation einer Nukleinsäure, die für ein Tumor-assoziiertes Antigen kodiert, umfassen beispielsweise ein Paar von Amplifikationsprimern, die an die Nukleinsäure hybridisieren, die für das Tumor-assoziierte Antigen kodiert. Die Primer umfassen vorzugsweise eine Sequenz von 6-50, insbesondere 10-30, 15-30 oder 20-30 zusammenhängenden Nukleotiden aus der Nukleinsäure und sind nicht-überlappend, um die Bildung von Primer-Dimeren zu vermeiden. Einer der Primer wird an einen Strang der Nukleinsäure hybridisieren, die für das Tumor-assoziierte Antigen kodiert, und der andere Primer wird an den komplementären Strang in einer Anordnung hybridisieren, die eine Amplifikation der Nukleinsäure, die für das Tumor-assoziierte Antigen kodiert, erlaubt.

Vorzugsweise besteht ein Oligonukleotid aus Ribonukleotiden, Desoxyribonukleotiden oder einer Kombination davon. Dabei sind das 5'-Ende eines Nukleotids und das 3'-Ende eines anderen Nukleotids durch eine Phosphodiesterbindung miteinander verknüpft. Diese Oligonukleotide können in herkömmlicher Weise synthetisiert oder rekombinant produziert werden.

Vorzugsweise ist ein Oligonukleotid ein "modifiziertes" Oligonukleotid. Dabei kann das Oligonukleotid, um beispielsweise seine Stabilität zu erhöhen, auf verschiedenste Art und Weise modifiziert sein ohne dass seine Fähigkeit, an sein Ziel zu binden, beeinträchtigt wird. Der Begriff "modifiziertes Oligonukleotid" bedeutet erfindungsgemäß ein Oligonukleotid, bei dem (i) mindestens zwei seiner Nukleotide durch eine synthetische Internukleosidbindung (d.h. eine Internukleosidbindung, die keine Phosphodiesterbindung ist) miteinander verknüpft sind und/oder (ii) eine chemische Gruppe kovalent mit dem Oligonukleotid verbunden ist, die normalerweise nicht bei Nukleinsäuren auftritt. Bevorzugte synthetische Internukleosidbindungen sind Phosphorothioate, Alkylphosphonate, Phosphorodithioate, Phosphatester, Alkylphosphonothioate, Phosphoramidate, Carbamate, Carbonate, Phosphattriester, Acetamidate, Carboxymethylester und Peptide.

Der Begriff "modifiziertes Oligonukleotid" umfasst auch Oligonukleotide mit einer kovalent modifizierten Base und/oder Zucker. "Modifizierte Oligonukleotide" umfassen beispielsweise Oligonukleotide mit Zuckerresten, die kovalent an organische Gruppen mit einem geringen Molekulargewicht gebunden sind, die keine Hydroxylgruppe an der 3'-Position und keine Phosphatgruppe an der 5'-Position sind. Modifizierte Oligonukleotide können beispielsweise einen 2'-O-alkylierten Riboserest oder einen anderen Zucker anstelle von Ribose wie Arabinose umfassen.

Die erfindungsgemäß beschriebenen Proteine und Polypeptide sind vorzugsweise isoliert. Die Begriffe "isoliertes Protein" oder "isoliertes Polypeptid" bedeuten, dass das Protein oder Polypeptid von seiner natürlichen Umgebung getrennt ist. Ein isoliertes Protein oder Polypeptid kann in einem im Wesentlichen aufgereinigten Zustand vorliegen. Der Begriff "im wesentlichen aufgereinigt" bedeutet, dass das Protein oder Polypeptid im wesentlichen frei von anderen Substanzen vorliegt, mit denen es in der Natur oder *in vivo* vorliegt.

Solche Proteine und Polypeptide dienen beispielsweise der Herstellung von Antikörpern und sind in einem immunologischen oder diagnostischen Assay oder als Therapeutika einsetzbar. Erfindungsgemäß beschriebene Proteine und Polypeptide können aus biologischen Proben wie Gewebe- oder Zellhomogenaten isoliert werden und können auch rekombinant in einer Vielzahl pro- oder eukaryontischer Expressionssysteme exprimiert werden.

"Derivate" eines Proteins oder Polypeptids oder einer Aminosäuresequenz im Sinne dieser Erfindung umfassen Aminosäure-Insertionsvarianten, Aminosäure-Deletionsvarianten und/oder Aminosäure-Substitutionsvarianten.

Aminosäure-Insertionsvarianten umfassen amino- und/oder carboxyterminale Fusionen, sowie Insertionen von einzelnen oder mehreren Aminosäuren in einer bestimmten Aminosäuresequenz. Bei Aminosäure-Sequenzvarianten mit einer Insertion werden ein oder mehrere Aminosäurereste in eine vorbestimmte Stelle in einer Aminosäuresequenz eingebracht, obwohl eine zufällige Insertion mit geeignetem Screening des resultierenden Produkts auch möglich ist. Aminosäure-Deletionsvarianten sind durch das Entfernen von einer oder mehreren Aminosäuren aus der Sequenz charakterisiert. Aminosäure-Substitutionsvarianten zeichnen sich dadurch aus, dass wenigstens ein Rest in der Sequenz entfernt und ein anderer Rest an dessen Stelle eingefügt wird. Vorzugsweise befinden sich die Modifikationen an Positionen in der Aminosäuresequenz, die zwischen homologen Proteinen oder Polypeptiden nicht konserviert sind. Vorzugsweise werden Aminosäuren durch andere mit ähnlichen Eigenschaften, wie Hydrophobizität, Hydrophilizität, Elektronegativität, Volumen der Seitenkette und ähnliches, ersetzt (konservative Substitution). Konservative Substitutionen betreffen beispielsweise den Austausch einer Aminosäure durch eine andere, wobei beide Aminosäuren in derselben nachstehenden Gruppe aufgeführt sind:
1. kleine aliphatische, nicht-polare oder leicht-polare Reste: Ala, Ser, Thr (Pro, Gly)
2. negativ geladene Reste und ihre Amide: Asn, Asp, Glu, Gln
3. positiv geladene Reste: His, Arg, Lys
4. große aliphatische, nicht-polare Reste: Met, Leu, Ile, Val (Cys)
5. große aromatische Reste: Phe, Tyr, Trp.

Drei Reste sind aufgrund ihrer besonderen Rolle für die Proteinarchitektur in Klammern gesetzt. Gly ist der einzige Rest ohne eine Seitenkette und verleiht der Kette somit Flexibilität. Pro besitzt eine ungewöhnliche Geometrie, die die Kette stark einschränkt. Cys kann eine Disulfidbrücke bilden.

Die oben beschriebenen Aminosäure-Varianten können leicht mit Hilfe von bekannten Peptidsynthesetechniken wie z.B. durch "Solid Phase Synthesis" (Merrifield, 1964) und ähnliche Verfahren oder durch rekombinante DNA-Manipulation hergestellt werden. Techniken, um Substitutionsmutationen an vorbestimmten Stellen in DNA einzubringen, die eine bekannte oder teilweise bekannte Sequenz besitzt, sind gut bekannt und umfassen z.B. M13-Mutagenese. Die Manipulation von DNA-Sequenzen zur Herstellung von Proteinen mit Substitutionen, Insertionen oder Deletionen ist z.B. in Sambrook et. al. (1989) ausführlich beschrieben.

"Derivate" von Proteinen oder Polypeptiden umfassen erfindungsgemäß auch einzelne oder multiple Substitutionen, Deletionen und/oder Additionen jeglicher Moleküle, die mit dem Enzym assoziiert sind, wie Kohlenhydrate, Lipide und/oder Proteine oder Polypeptide. Ferner erstreckt sich der Begriff "Derivat" auch auf alle funktionellen chemischen Äquivalente der Proteine oder Polypeptide.

Ein Teil oder Fragment eines Tumor-assoziierten Antigens weist erfindungsgemäß eine funktionelle Eigenschaft des Polypeptids auf, aus dem es abgeleitet ist. Solche funktionellen Eigenschaften umfassen die Interaktion mit Antikörpern, die Interaktion mit anderen Polypeptiden oder Proteinen, die selektive Bindung von Nukleinsäuren und eine enzymatische Aktivität. Eine bedeutende Eigenschaft ist die Fähigkeit, einen Komplex mit HLA einzugehen und gegebenenfalls eine Immunreaktion zu erzeugen. Diese Immunreaktion kann auf Stimulation von cytotoxischen oder Helfer-T-Zellen beruhen. Vorzugsweise umfasst ein erfindungsgemäßer Teil oder Fragment eines Tumor-assoziierten Antigens eine Sequenz von mindestens 6, insbesondere mindestens 8, mindestens 10, mindestens 12, mindestens 15, mindestens 20, mindestens 30 oder mindestens 50 aufeinanderfolgenden Aminosäuren aus dem Tumor-assoziierten Antigen. Ein Teil oder Fragment eines Tumor-assoziierten Antigens ist vorzugsweise ein Teil des Tumor-assoziierten Antigens, der dem nicht-Transmembrananteil, insbesondere dem extrazellulären Anteil des Antigens entspricht oder davon umfasst wird.

Ein Teil oder ein Fragment einer Nukleinsäure, die für ein Tumor-assoziiertes Antigen kodiert, betrifft erfindungsgemäß den Teil der Nukleinsäure, der zumindest für das Tumor-assoziierte Antigen und/oder für einen Teil oder ein Fragment des Tumor-assoziierten Antigens wie vorstehend definiert kodiert. Vorzugsweise ist ein Teil oder Fragment einer Nukleinsäure, die für ein Tumor-assoziiertes Antigen kodiert, derjenige Teil, der dem offenen Leserahmen, insbesondere wie im Sequenzprotokoll angegeben entspricht.

Eine Bestimmung von Nukleinsäuren, die für Tumor-assoziierte Antigene kodieren, kann in herkömmlicher Weise erfolgen, einschließlich durch Polymerase-Kettenreaktion oder Hybridisierung mit einer markierten Sonde. Eine Bestimmung von Tumor-assoziierten Antigenen kann durch ein Screening von Patienten-Antiseren in Bezug auf eine Erkennung des Antigens erfolgen. Sie kann auch durch ein Screening von T-Zellen des Patienten auf Spezifität für das entsprechende Tumor-assoziierte Antigen erfolgen.

Stoffe wie Polypeptide, die an Tumor-assoziierte Antigene binden, können z.B. in Screening-Assays für einen Nachweis von Tumor-assoziierten Antigenen und Komplexen von Tumor-assoziierten Antigenen mit ihren Bindepartnern sowie bei einer Aufreinigung der Tumor-assoziierten Antigene und von Komplexen davon mit ihren Bindepartnern Verwendung finden. Solche Stoffe können auch für eine Hemmung der Aktivität Tumor-assoziierter Antigene beispielsweise durch Bindung an solche Antigene Verwendung finden.

Bindestoffe, die die Fähigkeit aufweisen, selektiv an Tumor-assoziierte Antigene zu binden, umfassen polyklonale und monoklonale Antikörper, die in herkömmlicher Weise hergestellt werden.

Es ist bekannt, dass nur ein kleiner Teil eines Antikörpermoleküls, das Paratop, an der Bindung des Antikörpers an sein Epitop beteiligt ist (vgl. Clark, W.R. (1986), The Experimental Foundations of Modern Immunology, Wiley & Sons, Inc., New York; Roitt, I. (1991), Essential Immunology, 7. Auflage, Blackwell Scientific Publications, Oxford). Die pFc'- und Fc-Regionen sind z.B. Effektoren der Komplementkaskade, sind jedoch nicht an der Antigenbindung beteiligt. Ein Antikörper, von dem die pFc'-Region enzymatisch abgespalten wurde oder der ohne die pFc'-Region hergestellt wurde, bezeichnet als F(ab')₂-Fragment, trägt beide Antigenbindestellen eines vollständigen Antikörpers. In ähnlicher Weise trägt ein Antikörper, von dem die Fc-Region enzymatisch abgespalten wurde oder der ohne die Fc-Region hergestellt wurde, bezeichnet als Fab-Fragment, eine Antigenbindestelle eines intakten Antikörpermoleküls. Des weiteren bestehen Fab-Fragmente aus einer kovalent gebundenen leichten Kette eines Antikörpers und einem Teil der schweren Kette des Antikörpers, bezeichnet als Fd. Die Fd-Fragmente sind die Haupt-Determinanten der Antikörper-Spezifität (ein einzelnes Fd-Fragment kann mit bis zu zehn verschiedenen leichten Ketten assoziiert werden, ohne die Spezifität des Antikörpers zu verändern) und Fd-Fragmente behalten bei einer Isolierung die Fähigkeit, an ein Epitop zu binden.

Innerhalb des Antigen-bindenden Teils eines Antikörpers befinden sich komplementaritätsbestimmende Regionen (CDRs), die direkt mit dem Epitop des Antigens wechselwirken, und Gerüstregionen (FRs), die die Tertiärstruktur des Paratops aufrechterhalten. Sowohl in dem Fd-Fragment der schweren Kette als auch in der leichten Kette von IgG-Immunglobulinen befinden sich vier Gerüstregionen (FR1 bis FR4), die jeweils durch drei komplementaritätsbestimmende Regionen (CDR1 bis CDR3) getrennt sind. Die CDRs und insbesondere die CDR3-Regionen und noch mehr die CDR3-Region der schweren Kette sind größtenteils für die Antikörper-Spezifität verantwortlich.

Man weiß, dass die Nicht-CDR-Regionen eines Säuger-Antikörpers durch ähnliche Regionen von Antikörpern mit der gleichen oder einer anderen Spezifität ersetzt werden können, wobei die Spezifität für das Epitop des ursprünglichen Antikörpers erhalten bleibt. Dies ermöglichte die Entwicklung sogenannter "humanisierter" Antikörper, bei denen nicht-menschliche CDRs kovalent mit menschlichen FR- und/oder Fc/pFc'-Regionen für die Herstellung eines funktionellen Antikörpers verbunden sind.

Zum Beispiel beschreibt die WO 92/04381 die Herstellung und Verwendung von humanisierten RSV-Antikörpern aus Maus, bei denen mindestens ein Teil der FR-Regionen aus Maus durch FR-Regionen eines menschlichen Ursprungs ersetzt wurden. Solche Antikörper, einschließlich Fragmente intakter Antikörper mit einer Antigen-Bindefähigkeit werden oft als "chimäre" Antikörper bezeichnet.

Erfindungsgemäß sind auch F(ab')₂-, Fab-, Fv- und Fd-Fragmente von Antikörpern, chimäre Antikörper, bei denen die Fc- und/oder FR- und/oder CDR1- und/oder CDR2- und/oder leichte Kette-CDR3-Regionen durch homologe menschliche oder nicht-menschliche Sequenzen ersetzt wurden, chimäre F(ab')₂-Fragment-Antikörper, bei denen die FR- und/oder CDR1- und/oder CDR2- und/oder leichte Kette-CDR3-Regionen durch homologe menschliche oder nicht-menschliche Sequenzen ersetzt wurden, chimäre Fab-Fragment-Antikörper, bei denen die FR- und/oder CDR1- und/oder CDR2- und/oder leichte Kette-CDR3-Regionen durch homologe menschliche oder nicht-menschliche Sequenzen ersetzt wurden, und chimäre Fd-Fragment-Antikörper, bei denen die FR- und/oder CDR1- und/oder CDR2-Regionen durch homologe menschliche oder nicht-menschliche Sequenzen ersetzt wurden, vorgesehen. Erfindungsgemäß vorgesehen sind auch sogenannte einzelkettige Antikörper.

Vorzugsweise ist ein erfindungsgemäß verwendeter Antikörper gegen eine der in SEQ ID NOs: 10, 17 und 18 dargestellten Sequenzen oder einen Teil oder ein Derivat davon gerichtet und/oder kann durch Immunisierung mit diesen Peptiden erhalten werden.

Polypeptide, die spezifisch an Tumor-assoziierte Antigene binden, können durch degenerierte Peptid-Bibliotheken bereitgestellt werden, die einfach in Lösung in einer immobilisierten Form oder als Phagen-Display-Bibliotheken hergestellt werden können. Kombinatorische Bibliotheken aus Peptiden mit einer oder mehreren Aminosäuren können ebenfalls hergestellt werden. Ferner können Bibliotheken aus Peptoiden und nicht-peptidischen synthetischen Resten hergestellt werden.

Phagen-Display kann besonders wirksam bei der Identifizierung von Bindepeptiden sein. Dabei wird beispielsweise eine Phagen-Bibliothek (durch Verwendung beispielsweise des m13-, fd- oder lambda-Phagen) hergestellt, die Inserts einer Länge von 4 bis etwa 80 Aminosäureresten präsentiert. Es werden sodann Phagen ausgewählt, die Inserts tragen, die an das Tumor-assoziierte Antigen binden. Dieser Prozess kann über mehrere Zyklen einer Rückselektion von Phagen wiederholt werden, die an das Tumor-assoziierte Antigen binden. Wiederholte Runden führen zu einer Anreicherung von Phagen, die bestimmte Sequenzen tragen. Es kann eine Analyse von DNA-Sequenzen erfolgen, um die Sequenzen der exprimierten Polypeptide zu identifizieren. Der kleinste lineare Anteil der Sequenz, der an das Tumor-assoziierte Antigen bindet, kann bestimmt werden. Das "two-hybrid-System" aus Hefe kann auch für die Identifizierung von Polypeptiden eingesetzt werden, die an ein Tumor-assoziiertes Antigen binden. Tumor-assoziierte Antigene oder Fragmente davon können für ein Screening von Peptid-Bibliotheken, einschließlich Phagen-Display-Bibliotheken, eingesetzt werden, um Peptid-Bindepartner der Tumor-assoziierten Antigene zu identifizieren und selektieren. Solche Moleküle können beispielsweise für Screening-Assays, Aufreinigungsprotokolle, für eine Interferenz mit der Funktion des Tumor-assoziierten Antigens und für andere Zwecke, die dem Fachmann bekannt sind, verwendet werden.

Die vorstehend beschriebenen Antikörper und andere Bindemoleküle können beispielsweise für die Identifizierung von Gewebe verwendet werden, das ein Tumor-assoziiertes Antigen exprimiert. Antikörper können auch an spezifische diagnostische Stoffe für eine Darstellung von Zellen und Geweben gekoppelt werden, die Tumor-assoziierte Antigene exprimieren. Sie können ferner an therapeutisch nützliche Stoffe gekoppelt werden. Diagnostische Stoffe umfassen in nicht begrenzender Weise Bariumsulfat, locetaminsäure, Iopansäure, Calcium-Ipodat, Natrium-Diatrizoat, Meglumin-Diatrizoat, Metrizamid, Natrium-Tyropanoat und Radiodiagnostika, einschließlich Positronen-Emitter wie Fluor-18 und Kohlenstoff-11, gamma-Emitter wie Iod-123, Technetium-99m, Iod-l31 und Indium-111, Nuklide für magnetische Kernresonanz wie Fluor und Gadolinium. Der Begriff "therapeutisch nützlicher Stoff" meint erfindungsgemäß jedes therapeutische Molekül, das wunschgemäß selektiv zu einer Zelle geführt wird, die ein oder mehrere Tumor-assoziierte Antigene exprimiert, einschließlich Antikrebsmittel, mit radioaktivem Iod versehene Verbindungen, Toxine, cytostatische oder cytolytische Arzneistoffe, usw. Antikrebsmittel umfassen beispielsweise Aminoglutethimid, Azathioprin, Bleomycinsulfat, Busulfan, Carmustin, Chlorambucil, Cisplatin, Cyclophosphamid, Cyclosporin, Cytarabin, Dacarbazin, Dactinomycin, Daunorubin, Doxorubicin, Taxol, Etoposid, Fluoruracil, Interferon-α, Lomustin, Mercaptopurin, Methotrexat, Mitotan, Procarbazin-HCl, Thioguanin, Vinblastinsulfat und Vincristinsulfat. Weitere Antikrebsmittel sind beispielsweise in Goodman und Gilman, "The Pharmacological Basis of Therapeutics", 8. Auflage, 1990, McGraw-Hill, Inc., insbesondere Kapitel 52 (Antineoplastic Agents (Paul Calabresi und Bruce A. Chabner)) beschrieben. Toxine können Proteine wie Pokeweed-antivirales Protein, Choleratoxin, Pertussistoxin, Ricin, Gelonin, Abrin, Diphtherie-Exotoxin oder *Pseudomonas-Exotoxin* sein. Toxinreste können auch Hochenergie-emittierende Radionuklide wie Kobalt-60 sein.

Der Begriff "Patient" bedeutet erfindungsgemäß Mensch, nicht menschlicher Primat oder ein anderes Tier, insbesondere Säugetier wie Kuh, Pferd, Schwein, Schaf, Ziege, Hund, Katze oder Nagetier wie Maus und Ratte. In einer besonders bevorzugten Ausführungsform ist der Patient ein Mensch.

"Abnormale Expression" bedeutet erfindungsgemäß, dass die Expression gegenüber dem Zustand bei einem gesunden Individuum verändert, vorzugsweise erhöht ist. Eine Erhöhung der Expression betrifft eine Erhöhung um mindestens 10%, insbesondere mindestens 20%, mindestens 50% oder mindestens 100%. In einer Ausführungsform wird das Tumor-assoziierte Antigen nur in Gewebe eines erkrankten Individuums exprimiert, während die Expression bei einem gesunden Individuum reprimiert ist.

Eine biologische Probe kann erfindungsgemäß eine Gewebe- und/oder zelluläre Probe sein und kann für eine Verwendung in den verschiedenen, hier beschriebenen Verfahren in herkömmlicher Weise gewonnen werden, wie durch Gewebebiopsie, einschließlich Stanzbiopsie, und Entnahme von Blut, Bronchialaspirat, Urin, Fäces oder anderen Körperflüssigkeiten.

Der Begriff "immunreaktive Zelle" bedeutet erfindungsgemäß eine Zelle, die in eine Immunzelle (wie B-Zelle, Helfer-T-Zelle oder cytolytische T-Zelle) bei geeigneter Stimulierung reifen kann. Immunreaktive Zellen umfassen CD34⁺ hämatopoietische Stammzellen, unreife und reife T-Zellen sowie unreife und reife B-Zellen. Falls die Herstellung cytolytischer oder Helfer-T-Zellen, die ein Tumor-assoziiertes Antigen erkennen, gewünscht ist, wird die immunreaktive Zelle mit einer Zelle, die ein Tumor-assoziiertes Antigen exprimiert, unter Bedingungen in Kontakt gebracht, die eine Produktion, Differenzierung und/oder Selektion von cytolytischen sowie Helfer-T-Zellen begünstigen. Die Differenzierung von T-Zell-Vorläufern in eine cytolytische T-Zelle bei einer Exposition gegenüber einem Antigen ist ähnlich zur klonalen Selektion des Immunsystems.

Manche therapeutische Verfahren beruhen auf einer Reaktion des Immunsystems eines Patienten, die zu einer Lyse Antigen-präsentierender Zellen führt, wie Krebszellen, die ein oder mehrere Tumor-assoziierte Antigene präsentieren. Dabei werden beispielsweise autologe cytotoxische T-Lymphozyten, die für einen Komplex aus einem Tumor-assoziierten Antigen und einem MHC-Molekül spezifisch sind, an einen Patienten mit einer Zellabnormalie verabreicht. Die Produktion solcher cytotoxischer T-Lymphozyten *in vitro* ist bekannt. Ein Beispiel für ein Verfahren zur Differenzierung von T-Zellen findet sich in der WO-A-96/33265. Im Allgemeinen wird eine Probe mit Zellen wie Blutzellen aus dem Patienten entnommen und die Zellen werden mit einer Zelle in Kontakt gebracht, die den Komplex präsentiert und eine Vermehrung von cytotoxischen T-Lymphozyten auslösen kann (z.B. dendritische Zellen). Die Zielzelle kann eine transfizierte Zelle wie eine COS-Zelle sein. Diese transfizierten Zellen präsentieren den gewünschten Komplex auf ihrer Oberfläche und stimulieren bei einer Kontaktierung mit cytotoxischen T-Lymphozyten deren Vermehrung. Die klonal expandierten autologen cytotoxischen T-Lymphozyten werden sodann an den Patienten verabreicht.

Bei einem anderen Verfahren zur Selektion Antigen-spezifischer cytotoxischer T-Lymphozyten werden fluorogene Tetramere von MHC-Klasse I-Molekül/Peptid-Komplexen für einen Nachweis spezifischer Klone von cytotoxischen T-Lymphozyten verwendet (Altman et al., Science 274:94-96, 1996; Dunbar et al., Curr. Biol. 8:413-416, 1998). Lösliche MHC-Klasse I-Moleküle werden *in vitro* in Gegenwart von β₂-Mikroglobulin und eines Peptid-Antigens, das an das Klasse I-Molekül bindet, gefaltet. Nach Aufreinigung der MHC/Peptid-Komplexe werden diese mit Biotin markiert. Tetramere werden durch Mischen der biotinylierten Peptid-MHC-Komplexe mit markiertem Avidin (z.B. Phycoerythrin) bei einem molaren Verhältnis von 4:1 gebildet. Tetramere werden sodann mit cytotoxischen T-Lymphozyten wie peripherem Blut oder Lymphknoten in Kontakt gebracht. Die Tetramere binden an cytotoxische T-Lymphozyten, die den Peptid-Antigen/MHC-Klasse I-Komplex erkennen. Zellen, die an die- Tetramere-gebunden werden, können durch Fluoreszenzgesteuerte Zellsortierung für eine Isolierung reaktiver cytotoxischer T-Lymphozyten sortiert werden. Die isolierten cytotoxischen T-Lymphozyten können sodann *in vitro* vermehrt werden.

Bei einem therapeutischen Verfahren, das als adoptiver Transfer bezeichnet wird (Greenberg, J. Immunol. 136(5):1917, 1986; Riddel et al., Science 257:238, 1992; Lynch et al., Eur. J. Immunol. 21:1403-1410, 1991; Kast et al., Cell 59:603-614, 1989), werden Zellen, die den gewünschten Komplex präsentieren (z.B. dendritische Zellen), mit cytotoxischen T-Lymphozyten des zu behandelnden Patienten kombiniert, was zu einer Vermehrung spezifischer cytotoxischer T-Lymphozyten führt. Die vermehrten cytotoxischen T-Lymphozyten werden sodann an einen Patienten mit einer zellulären Abnormalie verabreicht, die sich durch bestimmte abnormale Zellen auszeichnet, die den spezifischen Komplex präsentieren. Die cytotoxischen T-Lymphozyten lysieren sodann die abnormalen Zellen, wodurch eine gewünschte therapeutische Wirkung erreicht wird.

Oft lassen sich aus dem T-Zell-Repertoire eines Patienten lediglich niedrig-affine T-Zellen gegen einen solchen spezifischen Komplex vermehren, da die hochaffinen durch Toleranzentwicklung ausgelöscht worden sind. Eine Alternative kann hier ein Transfer des T-Zell-Rezeptors selbst sein. Hierfür werden ebenfalls Zellen, die den gewünschten Komplex präsentieren (z.B. dendritische Zellen), mit cytotoxischen T-Lymphozyten von Gesunden kombiniert. Dies führt zu einer Vermehrung hochaffiner spezifischer cytotoxischer T-Lymphozyten, wenn der Spender mit dem spezifischen Komplex bisher keinen Kontakt hatte. Der hochaffine T-Zell-Rezeptor aus diesen vermehrten spezifischen T-Lymphozyten wird kloniert und kann durch Gentransfer z.B. mit retroviralen Vektoren beliebig in T-Zellen von anderen Patienten transduziert werden. Adoptiver Transfer erfolgt dann mit diesen genetisch veränderten T-Lymphozyten (Stanislawski et al., Nat. Immunol. 2:962-70, 2001 ; Kessels et al., Nat. Immunol. 2:957-61, 2001).

Die vorstehenden therapeutischen Aspekte gehen davon aus, dass zumindest manche der abnormalen Zellen des Patienten einen Komplex aus einem Tumor-assoziierten Antigen und einem HLA-Molekül präsentieren. Eine Identifizierung solcher Zellen kann in an sich bekannter Weise erfolgen. Sobald Zellen, die den Komplex präsentieren, identifiziert wurden, können sie mit einer Probe aus dem Patienten, die cytotoxische T-Lymphozyten enthält, kombiniert-werden. Falls die Zellen, die den Komplex präsentieren, durch die cytotoxischen T-Lymphozyten lysiert werden, kann angenommen werden, dass ein Tumor-assoziiertes Antigen präsentiert wird.

Der adoptive Transfer ist nicht die einzige Therapieform, die anwendbar ist. Cytotoxische T-Lymphozyten können auch *in vivo* in an sich bekannter Weise erzeugt werden. Bei einem Verfahren werden nicht-proliferative Zellen verwendet, die den Komplex exprimieren. Die Zellen, die dabei verwendet werden, werden diejenigen sein, die normalerweise den Komplex exprimieren, wie bestrahlte Tumorzellen oder Zellen, die mit einem oder beiden Genen transfiziert wurden, die für eine Präsentation des Komplexes notwendig sind (d.h. das Antigen-Peptid und das präsentierende HLA-Molekül). Verschiedene Zelltypen können eingesetzt werden. Des weiteren können Vektoren verwendet werden, die eines oder beide der interessierenden Gene tragen. Virale oder bakterielle Vektoren sind besonders bevorzugt. Zum Beispiel können Nukleinsäuren, die für ein Tumor-assoziiertes Antigen oder einen Teil davon kodieren, funktionell mit Promotor- und Enhancersequenzen verknüpft werden, die eine Expression des Tumor-assoziierten Antigens oder eines Fragments davon in bestimmten Geweben oder Zelltypen steuern. Die Nukleinsäure kann in einen Expressionsvektor eingebaut werden. Expressionsvektoren können nicht-modifizierte extrachromosomale Nukleinsäuren, Plasmide oder virale Genome sein, in die eine Insertion exogener Nukleinsäuren möglich ist. Nukleinsäuren, die für ein Tumor-assoziiertes Antigen kodieren, können auch in ein retrovirales Genom inseriert werden, wodurch die Integration der Nukleinsäure in das Genom des Zielgewebes oder der Zielzelle ermöglicht wird. Bei diesen Systemen trägt ein Mikroorganismus wie Vacciniavirus, Poxvirus, Herpes simplex-Virus, Retrovirus oder Adenovirus das interessierende Gen und "infiziert" de facto Wirtszellen. Eine weitere bevorzugte Form ist die Einbringung des Tumor-assoziierten Antigens in Form von rekombinanter RNA. Diese kann z.B. durch liposomalen Transfer oder durch Elektroporation in Zellen eingebracht werden. Die resultierenden Zellen präsentieren den interessierenden Komplex und werden von autologen cytotoxischen T-Lymphozyten erkannt, die sich sodann vermehren.

Eine ähnliche Wirkung kann durch Kombination des Tumor-assoziierten Antigens oder eines Fragments davon mit einem Adjuvans erreicht werden, um einen Einbau in Antigen-präsentierende Zellen *in vivo* zu ermöglichen. Das Tumor-assoziierte Antigen oder ein Fragment davon können als Protein, als DNA (z.B. innerhalb eines Vektors) oder als RNA repräsentiert sein. Das Tumor-assoziierte Antigen wird prozessiert, um einen Peptidpartner für das HLA-Molekül zu ergeben, während ein Fragment davon präsentiert werden kann, ohne dass eine weitere Prozessierung erforderlich ist. Letzteres ist insbesondere der Fall, wenn diese an HLA-Moleküle binden können. Verabreichungsformen, bei denen das Gesamt-Antigen *in vivo* von einer dendritischen Zelle prozessiert wird, sind bevorzugt, da hier auch Helfer-T-Zell-Antworten entstehen können. Eine effektive Immunantwort benötigt diese (Ossendorp et al., Immunol. Lett. 74:75-9, 2000; Ossendorp et al., J. Exp. Med. 187:693-702, 1998). Im allgemeinen kann eine wirksame Menge des Tumor-assoziierten Antigens an einen Patienten z.B. durch eine intradermale Injektion verabreicht werden. Die Injektion kann aber auch intranodal in einen Lymphknoten erfolgen (Maloy et al., Proc. Natl. Acad. Sci. USA 98:3299-303, 2001). Sie kann auch in Kombination mit Reagenzien erfolgen, die eine Aufnahme in dendritische Zellen erleichtern. Bevorzugte Tumor-assoziierte Antigene umfassen diejenigen, die mit allogenen Krebs-Antiseren oder mit T-Zellen vieler Krebs-Patienten reagieren. Von besonderem Interesse sind aber auch solche, gegen die keine spontanen Immunantworten vorbestehen. Gegen diese können nachweislich Immunantworten induziert werden, die Tumoren lysieren können (Keogh et al., J. Immunol. 167:787-96, 2001; Appella et al., Biomed. Pept. Proteins Nucleic Acids 1:177-84, 1995; Wentworth et al., Mol. Immunol. 32:603-12, 1995).

Die erfindungsgemäß beschriebenen pharmazeutischen Zusammensetzungen können auch als Vakzinen für die Immunisierung eingesetzt werden. Die Begriffe "Immunisierung" oder "Vakzinierung" bedeuten erfindungsgemäß eine Erhöhung oder Aktivierung einer Immunreaktion gegenüber einem Antigen. Tiermodelle können für ein Testen einer immunisierenden Wirkung gegenüber Krebs durch Verwendung eines Tumor-assoziierten Antigens oder einer dafür kodierenden Nukleinsäure eingesetzt werden. Zum Beispiel können menschliche Krebszellen in eine Maus für die Schaffung eines Tumors eingebracht werden und eine oder mehrere Nukleinsäuren, die für Tumor-assoziierte Antigene kodieren, können verabreicht werden. Die Wirkung auf die Krebszellen (beispielsweise Verringerung der Tumorgröße) kann als Maß für die Wirksamkeit einer Immunisierung durch die Nukleinsäure gemessen werden.

Als Teil der Zusammensetzung für eine Immunisierung werden eines oder mehrere Tumor-assoziierte Antigene oder stimulierende Fragmente davon mit einem oder mehreren Adjuvanzien für eine Induktion einer Immunreaktion oder eine Erhöhung einer Immunreaktion verabreicht. Ein Adjuvans ist eine Substanz, die in das Antigen eingebaut oder gemeinsam mit diesem verabreicht wird und die Immunreaktion verstärkt. Adjuvanzien können die Immunreaktion durch Bereitstellen eines Antigen-Reservoirs (extrazellulär oder in Makrophagen), Aktivierung von Makrophagen und Stimulierung bestimmter Lymphozyten verstärken. Adjuvanzien sind bekannt und umfassen in nicht begrenzender Weise Monophosphoryl-Lipid-A (MPL, SmithKline Beecham), Saponine wie QS21 (SmithKline Beecham), DQS21 (SmithKline Beecham; WO 96/33739), QS7, QS17, QS18 und QS-L1 (So et al., Mol. Cells 7:178-186, 1997), unvollständiges Freundsches Adjuvans, vollständiges Feundsches Adjuvans, Vitamin E, Montanid, Alaun, CpG-Oligonukleotide (vgl. Krieg et al., Nature 374:546-9, 1995) und verschiedene Wasser-in-Öl-Emulsionen, die aus biologisch abbaubaren Ölen wie Squalen und/oder Tocopherol hergestellt werden. Vorzugsweise werden die Peptide in einer Mischung mit DQS21/MPL verabreicht. Das Verhältnis von DQS21 zu MPL beträgt typischerweise etwa 1:10 bis 10:1, vorzugsweise etwa 1:5 bis 5:1 und insbesondere etwa 1:1. Für eine Verabreichung an den Menschen sind DQS21 und MPL typischerweise in einer Vakzine-Formulierung in einem Bereich von etwa 1 µg bis etwa 100 µg vorhanden.

Andere Stoffe, die eine Immunreaktion des Patienten stimulieren, können auch verabreicht werden. Zum Beispiel sind Zytokine bei einer Vakzinierung aufgrund ihrer regulatorischen Eigenschaften auf Lymphozyten verwendbar. Solche Zytokine umfassen z.B. Interleukin-12 (IL-12), von dem gezeigt wurde, dass es die schützenden Wirkungen von Vakzinen verstärkt (vgl. Science 268:1432-1434, 1995), GM-CSF und IL-18.

Es gibt eine Reihe von Verbindungen, die eine Immunreaktion verstärken und die daher bei einer Vakzinierung eingesetzt werden können. Diese umfassen co-stimulierende Moleküle, die in Form von Proteinen oder Nukleinsäuren bereitgestellt werden. Solche co-stimulierenden Moleküle sind beispielsweise B7-1 und B7-2 (CD80 bzw. CD86), die auf dendritischen Zellen (DC) exprimiert werden und mit dem auf den T-Zellen exprimierten CD28-Molekül interagieren. Diese Interaktion stellt eine Co-Stimulierung (Signal 2) für eine Antigen/MHC/TCR-stimulierte (Signal 1) T-Zelle bereit, wodurch die Vermehrung der T-Zelle und die Effektorfunktion verstärkt wird. B7 interagiert auch mit CTLA4 (CD152) auf T-Zellen und Untersuchungen, die CTLA4- und B7-Liganden einbeziehen, zeigen, dass die B7-CTLA4-Interaktion eine Antitumor-Immunität und CTL-Vermehrung verstärken kann (Zheng, P. et al., Proc. Natl. Acad. Sci. USA 95(11):6284-6289 (1998)).

B7 wird typischerweise nicht auf Tumorzellen exprimiert, so dass diese keine wirksamen Antigen-präsentierenden Zellen (APCs) für T-Zellen sind. Eine Induktion der B7-Expression würde ermöglichen, dass Tumorzellen wirksamer eine Vermehrung von cytotoxischen T-Lymphozyten und eine Effektorfunktion stimulieren. Eine Co-Stimulierung durch eine Kombination von B7/IL-6/IL-12 zeigte eine Induktion des IFN-gamma- und Th1-Zytokin-Profils in einer T-Zell-Population, was zu einer weiter verstärkten T-Zell-Aktivität führt (Gajewski et al., J. Immunol. 154:5637-5648 (1995)).

Eine vollständige Aktivierung von cytotoxischen T-Lymphozyten und eine vollständige Effektorfunktion erfordert eine Mitwirkung von T-Helferzellen durch die Interaktion zwischen dem CD40-Liganden auf den T-Helferzellen und dem CD40-Molekül, das von dendritischen Zellen exprimiert wird (Ridge et al., Nature 393:474 (1998), Bennett et al., Nature 393:478 (1998), Schönberger et al., Nature 393:480 (1998)). Der Mechanismus dieses co-stimulierenden Signals betrifft wahrscheinlich die Steigerung der B7- und assoziierten IL-6/IL-12-Produktion durch die dendritischen Zellen (Antigen-präsentierenden Zellen). Die CD40-CD40L-Interaktion komplementiert so die Interaktionen des Signals 1 (Antigen/MHC-TCR) und des Signals 2 (B7-CD28).

Die Verwendung von anti-CD40-Antikörpern für eine Stimulierung von dendritischen Zellen würde erwartungsgemäß direkt eine Reaktion gegenüber Tumor-Antigenen verstärken, die normalerweise außerhalb des Bereichs einer entzündlichen Reaktion liegen oder von nichtprofessionellen Antigen-präsentierenden Zellen (Tumorzellen) präsentiert werden. In diesen Situationen werden T-Helfer- und B7-co-stimulierende Signale nicht bereitgestellt. Dieser Mechanismus könnte im Zusammenhang mit Therapien verwendet werden, die auf Antigengepulsten dendritischen Zellen basieren, oder in Situationen, bei denen T-Helfer-Epitope nicht in bekannten TRA-Vorläufern definiert wurden.

Eine Verabreichung von Polypeptiden und Peptiden kann in an sich bekannter Weise erfolgen. In einer Ausführungsform erfolgt die Verabreichung von Nukleinsäuren durch ex vivo-Verfahren, d.h. durch Entfernung von Zellen aus einem Patienten, genetische Veränderung der Zellen, um ein Tumor-assoziiertes Antigen einzubauen, und Wiedereinbringung der veränderten Zellen in den Patienten. Dies umfasst im Allgemeinen das Einbringen einer funktionellen Kopie eines Gens in die Zellen eines Patienten *in vitro* und die Rückführung der genetisch veränderten Zellen in den Patienten. Die funktionelle Kopie des Gens steht unter funktioneller Kontrolle von regulatorischen Elementen, die eine Expression des Gens in den genetisch veränderten Zellen erlauben. Transfektions- und Transduktionsverfahren sind dem Fachmann bekannt. Erfindungsgemäß vorgesehen ist auch eine Verabreichung von Nukleinsäuren *in vivo* durch die Verwendung von Vektoren wie Viren und zielgesteuerten Liposomen.

In einer bevorzugten Ausführungsform ist ein viraler Vektor für die Verabreichung einer Nukleinsäure, die für ein Tumor-assoziiertes Antigen kodiert, aus der Gruppe ausgewählt bestehend aus Adenoviren, Adeno-assoziierten Viren, Poxviren, einschließlich Vacciniavirus und attenuierten Poxviren, Semliki-Forest-Virus, Retroviren, Sindbis-Virus und Ty-Virus-ähnlichen Partikeln. Besonders bevorzugt sind Adenoviren und Retroviren. Die Retroviren sind üblicherweise replikationsdefizient (d.h. sie sind unfähig, infektiöse Partikel zu erzeugen).

Verschiedene Verfahren können eingesetzt werden, um erfindungsgemäß Nukleinsäuren in Zellen *in vitro* oder *in vivo* einzubringen. Solche Verfahren umfassen die Transfektion von Nukleinsäure-Kalziumphosphat-Präzipitaten, die Transfektion von Nukleinsäuren, die mit DEAE assoziiert sind, die Transfektion oder Infektion mit den vorstehenden Viren, die die interessierenden Nukleinsäuren tragen, die Liposomen-vermittelte Transfektion und ähnliches. In bestimmten Ausführungsformen ist eine Steuerung der Nukleinsäure an bestimmte Zellen bevorzugt. In solchen Ausführungsformen kann ein Träger, der für die Verabreichung einer Nukleinsäure an eine Zelle (z.B. ein Retrovirus oder ein Liposom) eingesetzt wird, ein gebundenes Zielsteuerungsmolekül aufweisen. Zum Beispiel kann ein Molekül wie ein Antikörper, der für ein Oberflächenmembran-Protein auf der Zielzelle spezifisch ist, oder ein Ligand für einen Rezeptor auf der Zielzelle in den Nukleinsäureträger eingebaut oder daran gebunden werden. Bevorzugte Antikörper umfassen Antikörper, die selektiv ein Tumor-assoziiertes Antigen binden. Falls eine Verabreichung einer Nukleinsäure durch Liposomen erwünscht ist, können Proteine, die an ein Oberflächenmembran-Protein binden, das mit der Endozytose assoziiert ist, in die Liposomenformulierung eingebaut werden, um eine Zielsteuerung und/oder Aufnahme zu ermöglichen. Solche Proteine umfassen Kapsid-Proteine oder Fragmente davon, die für einen bestimmten Zelltyp spezifisch sind, Antikörper gegen Proteine, die internalisiert werden, Proteine, die eine intrazelluläre Stelle ansteuern, und ähnliches.

Die erfindungsgemäßen therapeutischen Zusammensetzungen können in pharmazeutisch verträglichen Zubereitungen verabreicht werden. Solche Zubereitungen können gewöhnlich pharmazeutisch verträgliche Konzentrationen von Salzen, Pufferstoffen, Konservierungsstoffen, Trägern, ergänzenden immunitätssteigernden Stoffen wie Adjuvanzien (z.B. CpG-Oligonukleotide) und Zytokine und gegebenenfalls andere therapeutische Wirkstoffe enthalten.

Therapeutische Wirkstoffe können auf jedem herkömmlichen Weg verabreicht werden, einschließlich durch Injektion oder durch Infusion. Die Verabreichung kann beispielsweise oral, intravenös, intraperitoneal, intramuskulär, subkutan oder transdermal erfolgen. Eine therapeutische Verabreichung von Antikörpern erfolgt vorzugsweise durch ein Lungenaerosol.

Die erfindungsgemäßen Zusammensetzungen werden in wirksamen Mengen verabreicht. Eine "wirksame Menge" betrifft die Menge, die alleine oder zusammen mit weiteren Dosen eine gewünschte Reaktion oder eine gewünschte Wirkung erzielt. Im Fall einer Behandlung einer bestimmten Erkrankung oder eines bestimmten Zustands, der sich durch die Expression eines oder mehrerer Tumor-assoziierter Antigene auszeichnet, betrifft die gewünschte Reaktion die Hemmung des Krankheitsverlaufs. Dies umfasst die Verlangsamung des Fortschreitens der Erkrankung und insbesondere eine Unterbrechung des Fortschreitens der Erkrankung. Die gewünschte Reaktion bei einer Behandlung einer Erkrankung oder eines Zustands kann auch die Verzögerung des Ausbruchs oder eine Verhinderung des Ausbruchs der Erkrankung oder des Zustands sein.

Eine wirksame Menge einer erfindungsgemäßen Zusammensetzung wird von dem zu behandelnden Zustand, der Schwere der Krankheit, den individuellen Parametern des Patienten, einschließlich Alter, physiologischer Zustand, Größe und Gewicht, der Dauer der Behandlung, der Art einer begleitenden Therapie (falls vorhanden), dem spezifischen Verabreichungsweg und ähnlichen Faktoren abhängen.

Die erfindungsgemäßen pharmazeutischen Zusammensetzungen sind vorzugsweise steril und enthalten eine wirksame Menge der therapeutisch wirksamen Substanz für die Erzeugung der gewünschten Reaktion oder der gewünschten Wirkung.

Die Dosen der erfindungsgemäßen Zusammensetzungen, die verabreicht werden, können von verschiedenen Parametern wie der Verabreichungsart, dem Zustand des Patienten, dem gewünschten Verabreichungszeitraum, usw. abhängen. Für den Fall, dass eine Reaktion bei einem Patienten bei einer anfänglichen Dosis unzureichend ist, können höhere Dosen (oder effektiv höhere Dosen, die durch einen anderen, stärker lokalisierten Verabreichungsweg erzielt werden) eingesetzt werden.

Im Allgemeinen werden für eine Behandlung oder für eine Erzeugung oder Erhöhung einer Immunreaktion Dosen des Tumor-assoziierten Antigens von 1 ng bis 1 mg, vorzugsweise von 10 ng bis 100 µg formuliert und verabreicht. Falls die Verabreichung von Nukleinsäuren (DNA sowie RNA), die für Tumor-assoziierte Antigene kodieren, erwünscht ist, werden Dosen von 1 ng bis 0,1 mg formuliert und verabreicht.

Die erfindungsgemäßen pharmazeutischen Zusammensetzungen werden im Allgemeinen in pharmazeutisch verträglichen Mengen und in pharmazeutisch verträglichen Zusammensetzungen verabreicht. Der Begriff "pharmazeutisch verträglich" betrifft ein nichttoxisches Material, das nicht mit der Wirkung des aktiven Bestandteils der pharmazeutischen Zusammensetzung wechselwirkt. Solche Zubereitungen können gewöhnlich Salze, Pufferstoffe, Konservierungsstoffe, Träger und gegebenenfalls andere therapeutische Wirkstoffe enthalten. Bei einer Verwendung in der Medizin sollten die Salze pharmazeutisch verträglich sein. Nicht-pharmazeutisch verträgliche Salze können jedoch für die Herstellung pharmazeutisch verträglicher Salze davon verwendet werden und sind erfindungsgemäß umfasst. Solche pharmakologisch und pharmazeutisch verträglichen Salze umfassen in nicht begrenzender Weise diejenigen, die aus den nachstehenden Säuren hergestellt werden: Chlorwasserstoff-, Bromwasserstoff-, Schwefel-, Salpeter-, Phosphor-, Malein-, Essig-, Salicyl-, Citronen-, Ameisen-, Malon-, Bernsteinsäure und ähnliches. Pharmazeutisch verträgliche Salze können auch als Alkalimetall- oder Erdalkalimetallsalze wie Natrium-, Kalium- oder Calciumsalze hergestellt werden.

Eine erfindungsgemäße pharmazeutische Zusammensetzung kann einen pharmazeutisch verträglichen Träger umfassen. Der Begriff "pharmazeutisch verträglicher Träger" betrifft erfindungsgemäß einen oder mehrere kompatible feste oder flüssige Füllstoffe, Verdünnungsmittel oder Kapselsubstanzen, die für eine Verabreichung an einen Menschen geeignet sind. Der Begriff "Träger" betrifft einen organischen oder anorganischen Bestandteil, natürlicher oder synthetischer Natur, in dem der aktive Bestandteil kombiniert wird, um eine Anwendung zu erleichtern. Die Bestandteile der erfindungsgemäßen pharmazeutischen Zusammensetzung sind gewöhnlich derart, dass keine Interaktion auftritt, die die gewünschte pharmazeutische Wirksamkeit wesentlich beeinträchtigt.

Die erfindungsgemäßen pharmazeutischen Zusammensetzungen können geeignete Pufferstoffe wie Essigsäure in einem Salz, Citronensäure in einem Salz, Borsäure in einem Salz und Phosphorsäure in einem Salz enthalten.

Die pharmazeutischen Zusammensetzungen können auch gegebenenfalls geeignete Konservierungsstoffe wie Benzalkoniumchlorid, Chlorbutanol, Parabene und Thimerosal enthalten.

Die pharmazeutischen Zusammensetzungen werden gewöhnlich in einer einheitlichen Dosisform dargeboten und können in an sich bekannter Weise hergestellt werden. Erfindungsgemäße pharmazeutische Zusammensetzungen können beispielsweise in Form von Kapseln, Tabletten, Lutschpastillen, Lösungen, Suspensionen, Sirupen, Elixieren oder als Emulsion vorliegen.

Zusammensetzungen, die für eine parenterale Verabreichung geeignet sind, umfassen gewöhnlich eine sterile wässrige oder nicht-wässrige Zubereitung des Wirkstoffs, die vorzugsweise mit dem Blut des Empfängers isotonisch ist. Verträgliche Träger und Lösungsmittel sind beispielsweise Ringer-Lösung und isotonische Natriumchloridlösung. Zusätzlich werden gewöhnlich sterile, fixierte Öle als Lösungs- oder Suspensionsmedium eingesetzt.

Die vorliegende Erfindung wird durch die nachstehenden Abbildungen und Beispiele ausführlich beschrieben, die ausschließlich der Erläuterung dienen und nicht begrenzend zu verstehen sind. Dem Fachmann sind aufgrund der Beschreibung und der Beispiele weitere Ausführungsformen zugänglich, die ebenfalls erfindungsgemäß umfasst sind.

### Abbildungen

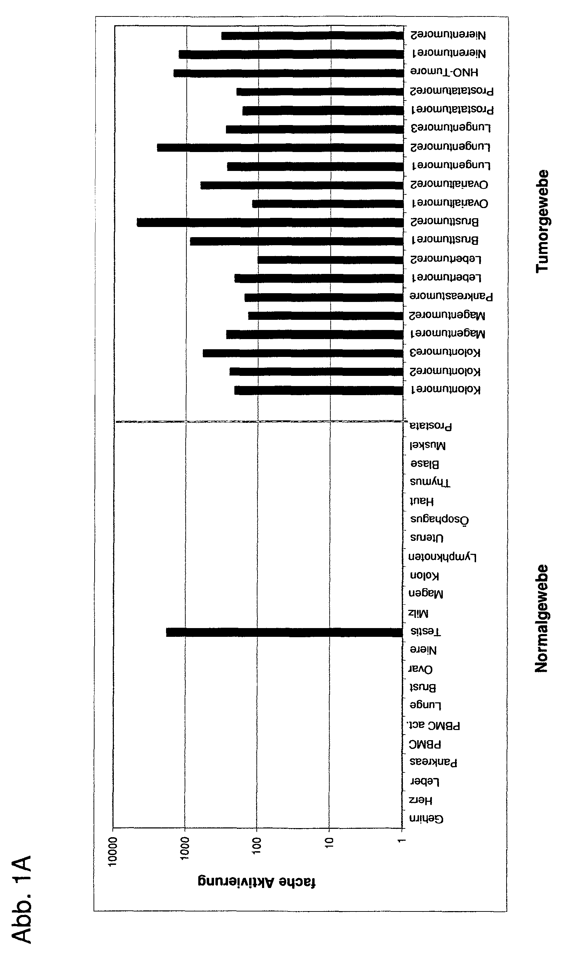

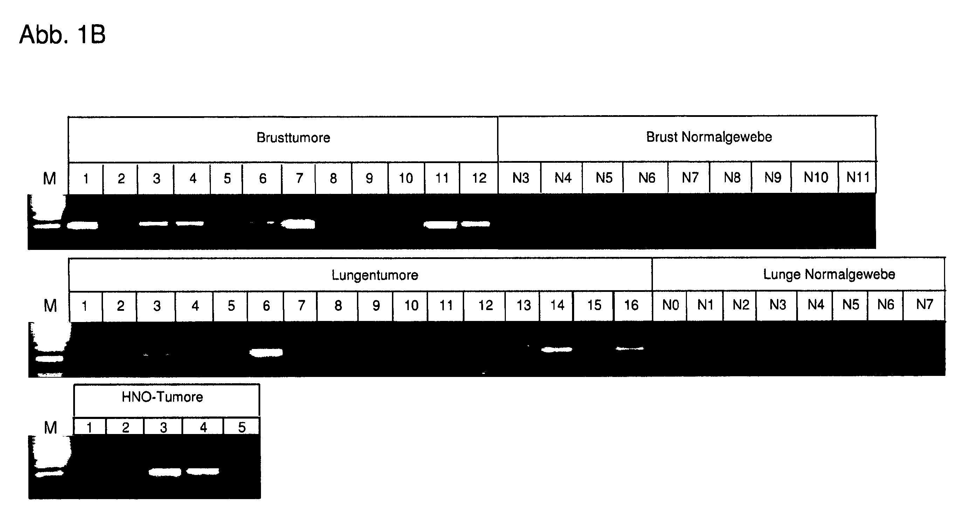

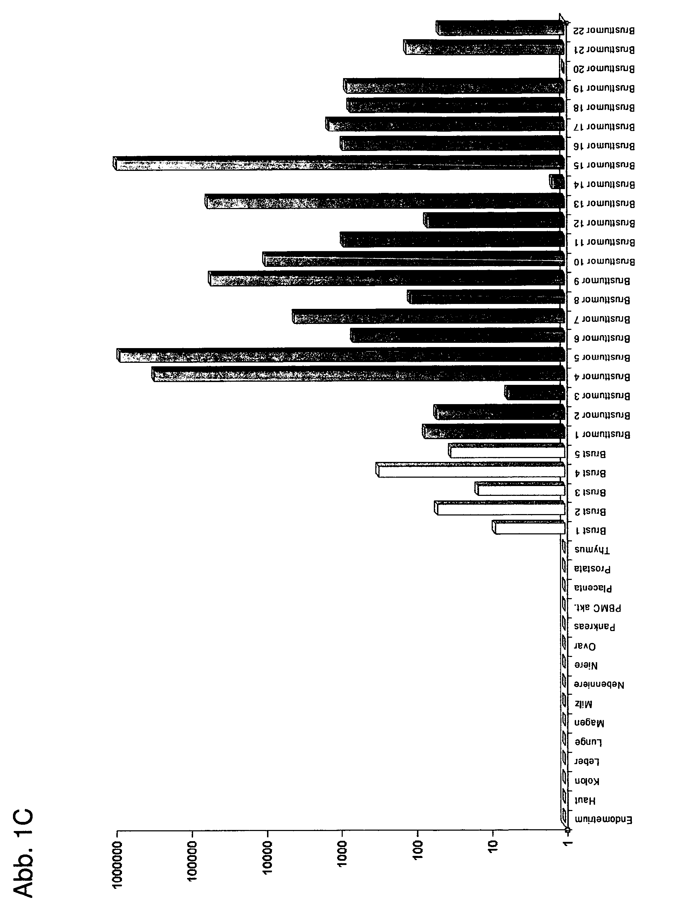

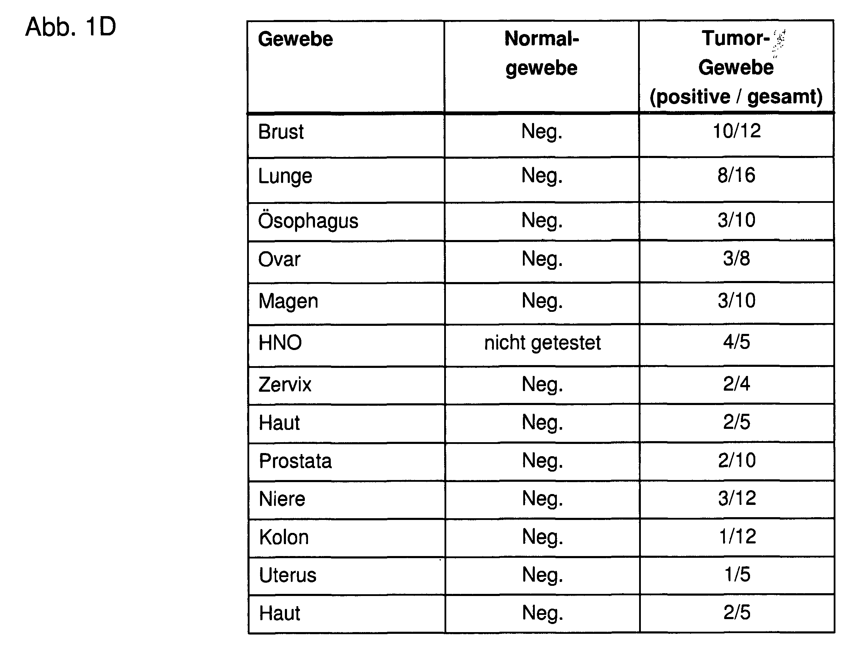

### Abb. 1: PCR-Analyse des Gens FLJ31461

**A:** Quantitative Expressionsanalyse von FLJ31461 in Normalgeweben (linke Seite) und in verschiedenen Tumoren (Pools bestehend aus jeweils 3-4 Einzelproben, rechte Seite) in logarithmischer Darstellung der relativen Expression (-fache Aktivierung). In den meisten Tumoren findet sich eine mindestens 100-fache Überexpression von FLJ31461 im Vergleich zu den Expressionsspiegeln in den gesunden Geweben.
**B**: Gelbild einer konventionellen RT-PCR-Analyse von FLJ31461 in Brust-, Lungen- und HNO-Tumoren mit den zugehörigen Normalgeweben Nₓ; M: DNA-Längenmarker.
**C**: Quantitative Expressionsanalyse in verschiedenen Normalgeweben (linke Seite) und in Brusttumoren in logarithmischer Darstellung der relativen Expression (-fache Aktivierung). In fast allen Brusttumoren findet sich eine mindestens 100-fache Überexpression von FLJ31461 im Vergleich zu den Expressionsspiegeln in den gesunden Geweben.
**D**: Zusammenfassung der FLJ31461-spezifischen Expression in verschiedenen analysierten Tumoren. Angegeben ist die Anzahl positiv getesteter Tumorproben/Gesamtzahl der analysierten Tumorproben. Während alle untersuchten somatischen Normalgewebe (jeweils 3-10 Gewebe, je nach Gewebstyp) keine Expression von FLJ31461 aufweisen, ist das Gen in sehr vielen Tumoren mit unterschiedlicher Häufigkeit exprimiert.

### Abb. 2: Protein-Lokalisation

Darstellung der zellulären Lokalisation des FLJ31461-Proteins. Die Abbildung zeigt die endogene Proteinexpression der Brusttumor-Zelllinie MCF7.

### Abb. 3: Immunhistochemische Analyse

**A**: Normalgewebe Testis (positive Membranlokalisation), Kolon und Niere (negative Membranlokalisation).
**B**: Nachweis des FLJ31461-Proteins in einem Bronchialkarzinom, einem Zervixkarzinom sowie einer Lymphknoten-Metastase eines Brusttumors in Übersicht (linke Spalte) und im Detail (rechte Spalte).
**C**: Zusammenfassung der immunhistochemischen Analysen des FLJ31461-Proteins. Angegeben ist die Anzahl positiv getesteter Tumorproben/Gesamtzahl der analysierten Tumorproben. Während alle untersuchten somatischen Normalgewebe keine Expression von FLJ31461 aufweisen, ist das Protein in sehr vielen Tumoren mit unterschiedlicher Häufigkeit an der Zelloberfläche zu finden.

### Beispiele

### Material und Methoden

Die Begriffe *"in silico",* und "elektronisch" beziehen sich rein auf die Nutzung von auf Datenbanken beruhenden Verfahren, mit denen auch Labor-experimentelle Vorgänge simuliert werden können.

Alle anderen Begriffe und Termini sind, falls nicht explizit anders definiert, so verwendet, wie sie der Fachmann versteht. Die genannten Techniken und Verfahren erfolgen in an sich bekannter Weise und sind z.B. in Sambrook et al., Molecular Cloning: A Laboratory Manual, 2. Auflage (1989), Cold Spring Harbor Laboratory Press, Cold Spring Harbor, N.Y. beschrieben. Alle Verfahren, die die Verwendung von Kits und Reagenzien einschließen, sind entsprechend den Angaben der Hersteller durchgeführt.

### A. Datamining-basierte Strategie zur Identifizierung von Tumor-assoziierten Antigenen

Öffentliche humane Protein- und Nukleinsäuredatenbanken wurden im Hinblick auf krebsspezifische Antigene untersucht, die auf der Zelloberfläche zugänglich sind. Die Definition der dafür notwendigen Filterkriterien zusammen mit einer Hochdurchsatz-Methodik zur Analyse möglichst aller Proteine bildet den zentralen Bestandteil dieser Strategie.

Den Ausgangspunkt bildeten die validierten Proteineinträge (NP-) bzw. die korrespondierenden mRNAs (NM-) in der "RefSeq" Datenbank (Pruitt et al., Trends Genet. Jan; 16(1):44-47, 2000) des "National Center for Biotechnology Information" (NCBI). Dem Grundprinzip Gen zu mRNA zu Protein folgend wurden die Proteine zunächst auf das Vorhandensein einer oder mehrerer Transmembrandomänen hin untersucht. Hierbei kam das Proteinanalyseprogramm TMHMM Server v. 2.0 (Krogh et al., Journal of Molecular Biology 305(3):567-580, 2001) zum Einsatz, dessen Ergebnisse anschließend noch einmal mit dem Programm ALOM **2** (Nakai et al., Geriomics 14:897-911, 1992) verifiziert wurden. Die Prädiktion weiterer Signalsequenzen, die die intrazelluläre Lokalisation von Proteinen beeinflussen, wurde mit den Programmen PSORT II (Horton et al., Intelligent Systems for Molecular Biology 4: 109-115, 1996) und iPSORT (Bannai et al., Bioinformatics, 18(2) 298-305, 2002) durchgeführt. Aus der humanen NP-Fraktion mit insgesamt 19.110 Einträgen wurden auf diese Weise 4.634 Proteine filtriert.

Die korrespondierende mRNA jedes dieser 4.634 Proteine wurde anschließend einer Homologiesuche in der EST-Datenbank (Boguski et al., Nat. Genet. 4(4):332-333, 1993) des NCBI mit Hilfe des "BLAST" Algorithmus (Altschul et al., Nucleic Acids Res.25:3389-3402, 1997) unterzogen. Die Filterkriterien wurden bei dieser Suche mit einem e-Value < 10e-20 und einer minimalen Sequenzidentität von 93% so eingestellt, dass die daraus resultierenden Treffer mit sehr hoher Wahrscheinlichkeit nur aus der jeweiligen mRNA und nicht aus homologen Transkripten hervorgegangen sein können. Fast alle mRNAs erzielten dabei mindestens einen Treffer in der EST-Datenbank, wobei die Anzahl der Treffer in Einzelfällen mehr als 4.000 betrug.

Für jeden Einzelnen dieser validen Treffer wurde anschließend die gewebsspezifische Herkunft der zugrunde liegenden cDNA-Bibliothek sowie der Name der Bibliothek ermittelt. Die daraus resultierenden Gewebe wurden in vier verschiedene Gruppen eingeteilt, die von dispensiblen Organen (Gruppe 3) bis hin zu absolut lebensnotwendrgen Organen reichten (Gruppe 0). Eine weitere Gruppe 4 bildeten alle Proben, die aus Krebsgewebe gewonnen wurden. Die Verteilung der Treffer auf die fünf Gruppen wurde in einer Tabelle festgehalten, die nach dem besten Verhältnis der Summe der Gruppen 3 und 4 gegenüber der Summe der Gruppen 0-2 sortiert wurde. Dabei erreichten diejenigen mRNAs einen Spitzenplatz, deren EST-Treffer ausschließlich Krebsgewebe entstammten, gefolgt von denjenigen, die darüber hinaus noch in Geweben dispensibler Organe der Gruppe 3 zu finden sind. Ein weiteres Kriterium für die Aussagekraft dieser Verteilung bildete die Anzahl der unabhängigen cDNA-Bibliotheken, aus denen die ESTs gewonnen wurden, und wurde in einer eigenen Spalte in der Tabelle festgehalten.

Da es sich bei den im ersten. Ansatz ermittelten Transkripten und den korrespondierenden Proteinen zunächst um hypothetische Konstrukte handelt, wurden noch weitere Filterkriterien hinzugezogen, die die reale Existenz der mRNAs und damit auch der Proteine belegen sollten. Dazu wurde jede mRNA mit Hilfe des Programms "Spidey" (Wheelan et al., Genome Res. 11(11):1952-1957, 2001) mit dem vorhergesagten Genlocus verglichen. Nur diejenigen Transkripte, die mindestens einen Spleißvorgang aufweisen, d.h. die sich auf mindestens 2 Exons verteilen, wurden für weitergehende Analysen verwendet.

Die sequenzielle Anwendung aller genannten Filter führte zu Tumor-assoziierten Antigenen, die aufgrund einer vorhergesagten Transmembrandomäne und der damit verbundenen Topologie als von extrazellulär zugänglich anzusehen sind. Das aus den EST-Daten abgeleitete Expressionsprofil weist in allen Fällen auf eine krebsspezifische Expression hin, die sich höchstens noch auf dispensible Organe erstrecken kann.

### B. Validierungsstrategie der durch in silico-Analyse identifizierten Tumor-assoziierten Antigene

Zur Nutzung der Antigene für immuntherapeutische Zwecke (Antikörpertherapie mittels monoklonaler Antikörper, Vakzinierung, T-Zell-Rezeptor-vermittelte therapeutische Ansätze; vgl. EP-B-0 879 282) bei der Krebstherapie sowie für diagnostische Fragestellungen ist die Validierung der identifizierten Antigene von zentraler Bedeutung. Die Validierung erfolgt dabei durch Expressionsanalyse sowohl auf RNA- als auch auf Proteinebene.

### 1. Untersuchung der RNA-Expression

Die erste Charakterisierung der identifizierten Tumorantigene erfolgt mit Hilfe von RNA, die aus verschiedenen Geweben bzw. aus gewebespezifischen Zelllinien gewonnen wird. Weil das differentielle Expressionsmuster aus gesundem Gewebe im Vergleich zu Tumorgewebe eine entscheidende Bedeutung für die spätere therapeutische Anwendung hat, erfolgt die Charakterisierung der Zielgene bevorzugt mit Hilfe dieser Gewebeproben.

Die Isolierung von Gesamt-RNA aus nativen Gewebeproben oder aus Tumorzelllinien erfolgt mit Verfahren, die in der Molekularbiologie Standard sind. Zum Beispiel kann die Isolierung mit Hilfe des RNeasy Maxi Kits (Qiagen, Kat. Nr. 75162) nach Vorschrift durch den Hersteller erfolgen. Dieses Isolierungsverfahren beruht auf der Verwendung von Guanidiniumisothiocyanat als chaotropes Reagenz. Alternativ kann die Isolierung mit saurem Phenol durchgeführt werden (Chomczynski & Sacchi, Anal. Biochem. 162: 156-159, 1987). Nach Aufarbeitung des Gewebes mittels Guanidiniumisothiocyanat wird die RNA mit saurem Phenol extrahiert, anschließend die RNA mit Isopropanol gefällt und in DEPC-behandeltem Wasser aufgenommen.

2-4 µg der so isolierten RNA werden anschließend z.B. mittels Superscript II (Invitrogen) entsprechend dem Protokoll des Herstellers in cDNA umgeschrieben. Das Priming der cDNA-Synthese erfolgt dabei mit Hilfe von zufälligen Hexameren (z.B. Roche Diagnostics) nach Standardprotokollen des jeweiligen Herstellers. Zur Qualitätskontrolle werden die cDNAs mit Primern in 30 Zyklen amplifiziert, die spezifisch für das nur gering exprimierte p53 Gen sind. Nur p53-positive cDNA-Proben werden für die weiteren Reaktionsschritte verwendet.

Zur detaillierten Antigenanalyse wird auf Basis eines cDNA-Archivs, das aus verschiedenen Normal- und Tumorgeweben sowie aus Tumorzelllinien isoliert wurde, eine Expressionsanalyse mittels PCR bzw. quantitativer PCR (qPCR) durchgeführt. Dazu werden 0,5 µl cDNA aus dem obigen Ansatz mit einer DNA-Polymerase (z.B. 1 U HotStarTaq DNA-Polyinerase, Qiagen) analog den Protokollen des jeweiligen Herstellers amplifiziert (Gesamtvolumen des Ansatzes: 25-50 µl). Neben der Polymerase enthält der Amplifikationsansatz 0,3 mM dNTPs, Reaktionsbuffer (Endkonzentration 1 x, abhängig vom Hersteller der DNA-Polymerase) und je 0,3 mM des genspezifischen "sense"- und "anti-sense"-Primers.

Die spezifischen Primer des Zielgens werden, soweit möglich, so ausgewählt, dass sie in zwei unterschiedlichen Exons liegen und somit genomische Kontaminationen nicht zu falsch positiven Ergebnissen führen. Bei einer nicht quantitativen Endpunkt-PCR wird die cDNA typischerweise 15 Minuten bei 95°C inkubiert, um die DNA zu denaturieren und um das Hot-Start-Enzym zu aktivieren. Anschließend wird die DNA in 35 Zyklen amplifiziert (1 min 95°C, 1 min Primer-spezifische Hybridisierungstemperatur (ca. 55-65°C), 1 min 72°C zur Elongation der Amplifikate). 10 µl des PCR Ansatzes werden anschließend auf Agarosegelen aufgetragen und im elektrischen Feld aufgetrennt. Durch Färben mit Ethidiumbromid wird die DNA in den Gelen sichtbar gemacht und das Ergebnis der PCR durch ein Foto dokumentiert.

Alternativ zur konventionellen PCR kann die Expressionsanalyse eines Zielgens auch durch quantitative "real time"-PCR erfolgen. Zu dieser Analyse sind inzwischen verschiedene Analysesysteme erhältlich, die bekanntesten sind das ABI 7900HT Sequence detection system (Applied Biosystems), der iCycler (Biorad) sowie der Light cycler (Roche Diagnostics). Wie oben beschrieben wird ein spezifischer PCR-Ansatz einem Lauf in einem der "real time"-PCR-Geräte unterzogen. Durch Zusatz eines DNA-interkalierenden Farbstoffes (z.B. Ethidiumbromid, SYBRGreen) wird die neu synthetisierte DNA durch spezifische Lichtanregung (nach Angaben der Farbstoffhersteller) sichtbar gemacht. Durch eine Vielzahl von Messpunkten während der Amplifikation kann der gesamte Prozess verfolgt und eine quantitative Bestimmung der Nukleinsäurekonzentration des Zielgens durchgeführt werden. Die Normalisierung des PCR-Ansatzes erfolgt durch Messung eines "housekeeping Gens" (z.B. 18S RNA, ß-Actin, GAPDH). Alternative Strategien über Fluoreszenz-markierte DNA-Sonden erlauben ebenfalls die quantitative Bestimmung des Zielgens aus einer spezifischen Gewebeprobe (siehe TaqMan-Applikationen der Fa. Applied Biosystems).

### 2. Klonierung

Die Klonierung des gesamten Zielgens, die für die weitere Charakterisierung des Tumorantigens notwendig ist, erfolgt nach gängigen molekularbiologischen Verfahren (z.B. in "Current Protocols in Molecular Biology", John Wiley & Sons Ltd., Wiley InterScience). Zur Klonierung bzw. Sequenzanalyse des Zielgens wird dieses zunächst mit einer DNA-Polymerase mit "proof reading-Funktion" (z.B. pfu, Roche Diagnostics) amplifiziert. Das Amplifikat wird anschließend mit Standardverfahren in einen Klonierungsvektor ligiert. Positive Klone werden durch Sequenzanalyse identifiziert und anschließend mit Hilfe von Prädiktionsprogrammen und bekannten Algorithmen charakterisiert.

### 3. Gewinnung von Antikörpern

Die Charakterisierung der identifizierten Tumor-assoziierten Antigene erfolgt beispielsweise durch die Verwendung von Antikörpern. Ferner umfasst die Erfindung die diagnostische oder therapeutische Verwendung von Antikörpern. Dabei können Antikörper Proteine in nativem und/oder denaturiertem Zustand erkennen (Anderson et al., J. Immunol. 143: 1899-1904, 1989; Gardsvoll, J. Immunol. Methods 234: 107-116, 2000; Kayyem et al., Eur. J. Biochem. 208: 1-8, 1992; Spiller et al., J. Immunol. Methods 224: 51-60, 1999).

Antiseren, die spezifische Antikörper enthalten, die an das Zielprotein spezifisch binden, können über verschiedene Standardverfahren hergestellt werden; vgl. beispielsweise "Monoclonal Antibodies: A Practical Approach" von Philip Shepherd, Christopher Dean ISBN 0-19-963722-9, "Antibodies: A Laboratory Manual" von Ed Harlow, David Lane ISBN: 0879693142 und "Using Antibodies: A Laboratory Manual: Portable Protocol NO" von Edward Harlow, David Lane, Ed Harlow ISBN: 0879695447. Dabei ist auch möglich, affine und spezifische Antikörper zu generieren, die komplexe Membranproteine in ihrer nativen Form erkennen (Azorsa et al., J. Immunol. Methods 229: 35-48, 1999; Anderson et al., J. Immunol. 143: 1899-1904, 1989; Gardsvoll, J. Immunol. Methods. 234: 107-116, 2000). Dies ist vor allem für die Herstellung von therapeutischen Antikörpern von Bedeutung, hat aber auch für viele diagnostische Anwendungen eine hohe Bedeutung. Dazu kann sowohl mit dem gesamten Protein als auch mit extrazellulären Teilsequenzen immunisiert werden.

### Immunisierung und Gewinnung von polyklonalen Antikörpern

Verschiedenste Immunisierungsprotokolle sind publiziert. Eine Spezies (z.B. Kaninchen, Mäuse) wird durch eine erste Injektion des gewünschten Zielproteins immunisiert. Durch eine zweite oder dritte Immunisierung innerhalb eines definierten Zeitraums (ca. 2-4 Wochen nach der vorangegangenen Immunisierung) lässt sich die Immunantwort des Tieres gegen das Immunogen verstärken. Wiederum nach verschiedenen definierten Zeitabständen (1. Blutung nach 4 Wochen, anschließend alle 2-3 Wochen bis zu 5 Entnahmen) wird den Tieren Blut entnommen und Immunserum gewonnen. Die so entnommenen Immunseren enthalten polyklonale Antikörper, mit denen das Zielprotein im Western Blot, durch die Durchflusszytometrie, Immunfluoreszenz oder Immunhistochemie nachgewiesen und charakterisiert werden kann.

Die Immunisierung der Tiere erfolgt in der Regel über eines von vier gut etablierten Verfahren, wobei auch andere Verfahren existieren. Immunisiert werden kann dabei mit für das Zielprotein spezifischen Peptiden, dem gesamten Protein, mit extrazellulären Teilsequenzen eines Proteins, das experimentell oder über Prädiktionsprogramme identifiziert werden kann. Da die Prädiktionsprogramme nicht immer fehlerfrei arbeiten, wird u.U. auch mit zwei Domänen gearbeitet, die voneinander durch eine Transmembrandomäne getrennt sind. Eine der beiden Domänen muss dann extrazellulär sein, was dann experimentell belegt werden kann (siehe nachstehend). Die Durchführung einer Immunisierung wird als Service von verschiedenen Dienstleistern kommerziell angeboten.
(1) Im ersten Fall werden Peptide (Länge: 8-12 Aminosäuren) über *in vitro*-Verfahren synthetisiert (durch einen kommerziellen Service möglich) und diese Peptide zur Immunisierung verwendet. In der Regel erfolgen 3 Immunisierungen (z.B. mit einer Konzentration von 5-100 µg/Immunisierung).
(2) Alternativ kann die Immunisierung durch rekombinante Proteine erfolgen. Dazu wird die klonierte DNA des Zielgens in einen Expressionsvektor kloniert und das Zielprotein analog den Bedingungen des jeweiligen Herstellers (z.B. Roche Diagnostics, Invitrogen, Clontech, Qiagen) z.B. zellfrei *in vitro,* in Bakterien (z.B. *E. coli),* in Hefe (z.B. *S. pombe*)*,* in Insektenzellen oder in Säugetierzellen synthetisiert. Dabei ist auch die Synthese des Zielproteins mit Hilfe von viralen Expressionssystemen möglich (z.B. Baculovirus, Vacciniavirus, Adenovirus). Nach Synthese in einem der Systeme wird das Zielprotein aufgereinigt. Die Aufreinigung erfolgt dabei in der Regel über chromatographische Verfahren. Dabei können auch Proteine für die Immunisierung verwendet werden, die über einen molekularen Anker als Hilfsmittel zur Reinigung verfügen (z.B. His-Tag, Qiagen; FLAG-Tag, Roche Diagnostics; GST-Fusionsproteine). Eine Vielzahl von Protokollen befindet sich z.B. in den "Current Protocols in Molecular Biology", John Wiley & Sons Ltd., Wiley InterScience. Nach Reinigung des Zielproteins erfolgt eine Immunisierung wie vorstehend beschrieben.
(3) Falls eine Zelllinie zur Verfügung steht, die das gewünschte Protein endogen synthetisiert, kann auch diese Zelllinie direkt zur Herstellung des spezifischen Antiserums verwendet werden. Die Immunisierung erfolgt dabei in 1-3 Injektionen mit jeweils ca. 1-5 x 10⁷ Zellen.
(4) Die Immunisierung kann auch durch Injektion von DNA (DNA-Immunisierung) erfolgen. Dazu wird das Zielgen zunächst in einen Expressionsvektor kloniert, so dass die Zielsequenz unter der Kontrolle eines starken eukaryontischen Promotors steht (z.B. CMV-Promotor). Anschließend wird DNA (z.B. 1-10 µg pro Injektion) als Immunogen mit einer "gene gun" in stark durchblutete, kapillare Bereiche eines Organismus transferiert (z.B. Maus, Kaninchen). Die transferierte DNA wird von Zellen des Tieres aufgenommen, das Zielgen wird exprimiert und das Tier entwickelt schließlich eine Immunantwort gegen das Zielprotein (Jung et al., Mol. Cells 12: 41-49, 2001; Kasinrerk et al., Hybrid Hybridomics 21: 287-293, 2002).

### Gewinnung monoklonaler Antikörper

Monoklonale Antikörper werden traditionell mit Hilfe der Hybridoma-Technologie hergestellt (Technische Details: siehe "Monoclonal Antibodies: A Practical Approach" von Philip Shepherd, Christopher Dean ISBN 0-19-963722-9; "Antibodies: A Laboratory Manual" von Ed Harlow, David Lane ISBN: 0879693142, "Using Antibodies: A Laboratory Manual: Portable Protocol NO" von Edward Harlow, David Lane, Ed Harlow ISBN: 0879695447). Als ein neues Verfahren wird auch die so genannte "SLAM"-Technologie eingesetzt. Hierbei werden B-Zellen aus Vollblut isoliert und die Zellen monoklonalisiert. Anschließend wird der Überstand der vereinzelten B-Zelle auf ihre Antikörperspezifität hin analysiert. Im Gegensatz zur Hybridomatechnologie wird anschließend die variable Region des Antikörpergens durch eine Einzelzell-PCR amplifiziert und in einen geeigneten Vektor kloniert. Auf diese Art und Weise wird die Gewinnung von monoklonalen Antikörpern beschleunigt (de Wildt et al. J. Immunol. Methods 207:61-67, 1997).

### 4: Validierung der Targets mit proteinchemischen Verfahren unter Verwendung von Antikörpern

Mit den Antikörpern, die wie vorstehend beschrieben herstellbar sind, lassen sich eine Reihe von wichtigen Aussagen zu dem Zielprotein treffen. Im Einzelnen sind die nachstehenden Analysen zur Validierung des Zielproteins sinnvoll:

### Spezifität des Antikörpers

Um zu zeigen, dass ein Antikörper spezifisch nur an das gewünschte Zielprotein bindet, eignen sich am besten auf Zellkultur-basierende Tests mit anschließendem Western Blot (verschiedene Variationen sind z.B. in "Current Protocols in Proteinchemistry", John Wiley & Sons Ltd., Wiley InterScience, beschrieben). Für den Nachweis werden Zellen mit einer cDNA für das Zielprotein transfiziert, die unter Kontrolle eines starken eukaryontischen Promotors steht (z.B. Cytomegalievirus-Promotor; CMV). Zur Transfektion von Zelllinien mit DNA sind die verschiedensten Verfahren (z.B. Elektroporation, auf Liposomen basierende Transfektion, Kalziumphosphatpräzipitation) gut etabliert (z.B. Lemoine et al., Methods Mol. Biol. 75: 441-7, 1997). Alternativ können auch Zelllinien verwendet werden, die das Zielgen endogen exprimieren (Nachweis über Zielgen-spezifische RT-PCR). Zur Kontrolle werden im Experiment im Idealfall homologe Gene gleichzeitig transfiziert, um im folgenden Western Blot die Spezifität des analysierten Antikörpers nachweisen zu können.

Im anschließenden Western Blot werden Zellen aus Zellkultur oder Gewebeproben, die das Zielprotein enthalten könnten, in einer 1%igen SDS Lösung lysiert und die Proteine dabei denaturiert. Die Lysate werden auf 8-15%igen denaturierenden Polyacrylamidgelen (enthalten 1% SDS) der Größe nach elektrophoretisch aufgetrennt (SDS-Polyacrylamid Gelelektrophorese, SDS-PAGE). Anschließend werden die Proteine durch eines von mehreren Blotting-Verfahren (z.B. semi-dry Elektroblot; Biorad) auf eine spezifische Membran transferiert (z.B. Nitrozellulose, Schleicher & Schüll). Auf dieser Membran kann das gewünschte Protein sichtbar gemacht werden. Dazu wird die Membran zunächst mit dem Antikörper, der das Zielprotein erkennt (Verdünnung ca. 1:20-1:200, je nach Spezifität des Antikörpers), für 60 Minuten inkubiert. Nach einem Waschschritt wird die Membran mit einem zweiten, mit einem Marker (z.B. Enzyme wie Peroxidase oder alkalische Phosphatase) gekoppelten Antikörper inkubiert, der den ersten Antikörper erkennt. In einer Farb- oder chemilumineszenten Reaktion kann anschließend das Zielprotein auf der Membran sichtbar gemacht werden (z.B. ECL, Amersham Bioscience). Ein Antikörper mit einer hohen Spezifität für das Zielprotein sollte im Idealfall nur das gewünschte Protein selbst erkennen.

### Lokalisation des Zielproteins

Zur Bestätigung der im *"in silico*"-Ansatz identifizierten Membranlokalisation des Zielproteins werden verschiedene Verfahren verwendet. Ein wichtiges und gut etabliertes Verfahren unter Verwendung der vorstehend beschriebenen Antikörper ist die Immunfluoreszenz (IF). Dazu werden Zellen etablierter Zelllinien benutzt, die entweder das Zielprotein synthetisieren (Nachweis der RNA in der RT-PCR oder des Proteins im Western Blot) oder aber mit Plasmid-DNA transfiziert worden sind. Zur Transfektion von Zelllinien mit DNA sind die verschiedensten Verfahren (z.B. Elektroporation, auf Liposomen basierende Transfektion, Kalziumphosphatpräzipitation) gut etabliert (z.B. Lemoine et al., Methods Mol. Biol. 75: 441-7, 1997). Das transfizierte Plasmid kann bei der Immunfluoreszenz das unmodifizierte Protein kodieren oder aber auch unterschiedliche Aminosäuremarker an das Zielprotein koppeln. Die wichtigsten Marker sind z.B. das fluoreszierende "green fluorescent protein" (GFP) in seinen verschiedenen differentiell fluoreszierenden Formen, kurze Peptidsequenzen von 6-12 Aminosäuren, für die hoch affine und spezifische Antikörper zur Verfügung stehen, oder die kurze Aminosäuresequenz Cys-Cys-X-X-Cys-Cys, die über ihre Cysteine spezifische fluoreszierende Substanzen binden kann (Invitrogen). Zellen, die das Zielprotein synthetisieren, werden z.B. mit Paraformaldehyd oder Methanol fixiert. Anschließend können die Zellen bei Bedarf durch Inkubation mit Detergenzien (z.B. 0,2% Triton X-100) permeabilisiert werden. Anschließend werden die Zellen mit einem primären Antikörper inkubiert, der gegen das Zielprotein oder gegen einen der gekoppelten Marker gerichtet ist. Nach einem Waschschritt wird der Ansatz mit einem zweiten, mit einem fluoreszierenden Marker (z.B. Fluorescin, Texas Red; DAKO) gekoppelten Antikörper inkubiert, der an den ersten Antikörper bindet. Anschließend werden die so markierten Zellen mit Glycerin überschichtet und mit Hilfe eines Fluoreszenzmikroskops nach den Angaben des Herstellers analysiert. Spezifische Fluoreszenzemissionen werden dabei, abhängig von den eingesetzten Substanzen, durch spezifische Anregung erreicht. Die Analyse erlaubt in der Regel die sichere Lokalisation des Zielproteins, wobei zur Bestätigung der Antikörperqualität und des Zielproteins in Doppelfärbungen zusätzlich zum Zielprotein auch die gekoppelten Aminosäuremarker oder andere Markerproteine angefärbt werden, deren Lokalisation bereits in der Literatur beschrieben ist. Ein Sonderfall stellt das GFP und seine Derivate dar, die direkt angeregt werden können und selbst fluoreszieren. Die Membranpermeabilität, die durch den Einsatz von Detergenzien gesteuert werden kann, erlaubt in der Immunfluoreszenz die Demonstration, ob ein immunogenes Epitop innerhalb oder außerhalb der Zelle lokalisiert ist. Die Prädiktion der ausgewählten Proteine kann so experimentell untermauert werden. Alternativ kann der Nachweis von extrazellulären Domänen mittels Durchflusszytometrie erfolgen. Dazu werden Zellen unter nicht permeabilisierenden Bedingungen (z.B. mit PBS/Na-Azid/2% FCS/ 5 mM EDTA) fixiert und im Durchflusszytometer nach Angaben des Herstellers analysiert. Nur extrazelluläre Epitope können bei diesem Verfahren von dem zu analysierenden Antikörper erkannt werden. Im Unterschied zu Immunfluoreszenz kann durch Verwendung von z.B. Propidiumiodid oder Trypanblau zwischen toten und lebenden Zellen unterschieden werden und damit falsch positive Ergebnisse vermieden werden.

Ein weiterer wichtiger Nachweis erfolgt durch die Immunhistochemie (IHC) an spezifischen Gewebeproben. Ziel dieses Verfahrens ist es, die Lokalisation eines Proteins in einem funktionell intakten Gewebeverband zu identifizieren. Die IHC dient im einzelnen dazu, um (1) die Menge an Zielprotein in Tumor- und Normalgeweben abschätzen zu können, (2) zu analysieren, wie viele Zellen in Tumor- und gesundem Gewebe das Zielgen exprimieren, und (3) den Zelltyp in einem Gewebe (Tumor, gesunde Zellen) zu definieren, in dem das Zielprotein nachweisbar ist. Alternativ können die Proteinmengen eines Zielgens durch Gewebsimmunfluoreszenz mittels Digitalkamera und geeigneter Software (z.B. Tillvision, Till-photonics, Deutschland) quantifiziert werden. Die Technologie ist häufig publiziert worden, Details für Färbung und Mikroskopie sind daher z.B. "Diagnostic Immunohistochemistry" von David J., MD Dabbs ISBN: 0443065667 oder in "Microscopy, Immunohistochemistry, and Antigen Retrieval Methods: For Light and Electron Microscopy" ISBN: 0306467704 zu entnehmen. Zu beachten ist, dass aufgrund der Eigenschaften von Antikörpern unterschiedliche Protokolle verwendet werden müssen (nachstehend ist ein Beispiel beschrieben), um zu einem aussagekräftigen Ergebnis zu kommen.

In der Regel werden histologisch definierte Tumorgewebe und als Referenz vergleichbare gesunde Gewebe in der IHC eingesetzt. Als Positiv- und Negativkontrollen können dabei auch Zelllinien dienen, bei denen die Präsenz des Zielgens durch RT-PCR Analysen bekannt ist. Eine Hintergrundkontrolle ist immer mitzuführen.

Formalin-fixierte (ein anderes Fixierungsverfahren mit z.B. Methanol ist auch möglich) und in Paraffin eingebettete Gewebestücke mit einer Dicke von 4µm werden auf einem Glasträger aufgebracht und z.B. mit Xylol deparaffiniert. Die Proben werden mit TBS-T gewaschen und in Serum blockiert. Anschließend erfolgt die Inkubation mit dem ersten Antikörper (Verdünnung: 1:2 bis 1:2000) für 1-18 Stunden, wobei in der Regel affinitätsgereinigete Antikörper verwendet werden. Nach einem Waschschritt erfolgt eine ca. 30-60-minütige Inkubation mit einem zweiten Antikörper, der mit einer Alkalischen Phosphatase (alternativ: z.B. Peroxidase) gekoppelt und gegen den ersten Antikörper gerichtet ist. Anschließend erfolgt eine Farbreaktion unter Verwendung der Alkalischen Phosphatase (vgl. beispielsweise Shi et al., J. Histochem. Cytochem. 39: 741-748, 1991; Shin et al., Lab. Invest. 64: 693-702, 1991). Zum Nachweis der Antikörper-Spezifität kann die Reaktion durch vorherige Zugabe des Immunogens kompetitiert werden.

### Analyse von Proteinmodifikationen

Sekundäre Proteinmodifikationen wie zum Beispiel N- und O-Glykosylierungen oder Myristilierungen können die Zugänglichkeit von immunogenen Epitopen behindern oder sogar ganz verhindern und damit die Wirksamkeit von Antikörpertherapien in Frage stellen. Zudem konnte vielfach nachgewiesen werden, dass sich Art und Menge der sekundären Modifikationen in Normal- und Tumorgewebe unterscheiden (z.B. Durand & Seta, 2000; Clin. Chem. 46: 795-805; Hakomori, 1996; Cancer Res. 56: 5309-18). Die Analyse dieser Modifikationen ist daher essentiell für den Therapieerfolg eines Antikörpers. Potentielle Bindestellen lassen sich durch spezifische Algorithmen prädizieren.

Die Analyse von Proteinmodifikationen erfolgt in der Regel im Western Blot (siehe vorstehend). Vor allem Glykosylierungen, die in der Regel eine Größe von mehreren kDa haben, führen zu einer größeren Gesamtmasse des Zielproteins, die sich in der SDS-PAGE auftrennen lässt. Zum Nachweis von spezifischen O- und N-glykosidischen Bindungen werden Proteinlysate vor der Denaturierung durch SDS mit O- oder N-Glykosylasen inkubiert (nach Angaben des jeweiligen Herstellers, z.B. PNgase, Endoglykosidase F, Endoglykosidase H, Roche Diagnostics). Anschließend erfolgt ein Western Blot wie vorstehend beschrieben. Bei Verringerung der Größe eines Zielproteins kann so nach Inkubation mit einer Glykosidase eine spezifische Glykosylierung nachgewiesen und auf diesem Weg auch die Tumorspezifität einer Modifikation analysiert werden.

### Funktionsanalyse des Zielgens

Die Funktion des Zielmoleküls kann entscheidend für seinen therapeutischen Nutzen sein, so dass funktionelle Analysen ein wichtiger Baustein bei der Charakterisierung von therapeutisch nutzbaren Molekülen sind. Die Funktionsanalyse kann entweder in Zellen, in Zellkulturexperimenten oder aber in vivo mit Hilfe von Tiermodellen erfolgen. Dabei wird das Gen des Zielmoleküls entweder durch Mutation ausgeschaltet ("knockout") oder aber die Zielsequenz in die Zelle bzw. den Organismus eingefügt ("knockin"). Man kann so funktionelle Veränderungen im zellulären Kontext einerseits durch den Funktionsverlust des zu analysierenden Genes ("loss of function") analysieren. Im zweiten Fall lassen sich Veränderungen analysieren, die durch die Ergänzung des analysierten Genes verursacht werden ("gain of function").

### a. Funktionsanalyse in Zellen

Transfektion. Zur Analyse des "gain of function" muss das Gen des Zielmoleküls in die Zelle transferiert werden. Dazu werden Zellen mit einer DNA transfiziert, die die Synthese des Zielmoleküls erlauben. In der Regel steht das Gen des Zielmoleküls dabei unter Kontrolle eines starken eukaryontischen Promotors (z.B. Cytomegalievirus-Promotor; CMV). Zur Transfektion von Zelllinien mit DNA sind die verschiedensten Verfahren (z.B. Elektroporation, auf Liposomen basierende Transfektion, Kalziumphosphatpräzipitation) gut etabliert (z.B. Lemoine et al., Methods Mol. Biol. 75: 441-7, 1997). Das Gen kann dabei entweder ohne genomische Integration transient oder aber mit genomischer Integration nach Selektion mit z.B. Neomycin stabil synthetisiert werden.

RNA interference (siRNA). Eine Expressionsinhibition des Zielgens, die unter Umständen einen vollständigen Funktionsverlust des Zielmoleküls in Zellen induziert, kann durch die "RNA interference" (siRNA)-Technologie in Zellen erzeugt werden (Hannon, GJ. 2002. RNA interference. Nature 418: 244-51; Czauderna et al. 2003. Nucl. Acid Res. 31: 670-82). Dazu werden Zellen mit kurzen, ca. 20-25 Nukleotide langen, doppelsträngigen RNA-Molekülen transfiziert, die für das Zielmolekül spezifisch sind. Ein enzymatischer Prozess führt anschließend zum Abbau der spezifischen RNA des Zielgens, was in einer verminderten Expression des Zielproteins resultiert, und ermöglicht somit die funktionelle Analyse des Zielgens.

Zelllinien, die mittels Transfektion oder siRNA modifiziert wurden, können anschließend auf unterschiedliche Art und Weise analysiert werden. Nachstehend sind die geläufigsten Beispiele aufgeführt.

### 1. Proliferation

Eine Vielzahl von Verfahren ist zur Analyse der Zellproliferation etabliert und wird von verschiedenen Unternehmen kommerziell angeboten (z.B. Roche Diagnostics, Invitrogen, Details zu den Testverfahren sind in den zahlreichen Applikationsprotokollen beschrieben). Die Zellzahl in Zellkulturexperimenten lässt sich durch einfaches Auszählen oder durch kolometrische Tests ermitteln, die die metabolische Aktivität der Zellen messen (z.B. wst-1, Roche Diagnostics). Metabolische Testverfahren messen indirekt über enzymatische Marker die Zellzahl in einem Experiment. Direkt kann die Zellproliferation durch Analyse der DNA-Syntheserate z.B. durch Zugabe von Bromdesoxyuridin (BrdU) gemessen werden, der Nachweis des integrierten BrdU erfolgt über spezifische Antikörper kolorimetrisch.

### 2. Apoptose und Zytotoxizität

Eine große Anzahl von Testsystemen zum Nachweis von zellulärer Apoptose und von Zytotoxizität ist verfügbar. Ein entscheidendes Charakteristikum ist die spezifische, enzymabhängige Fragmentierung der genomischen DNA, die irreversibel ist und sicher zum Tod der Zelle führt. Verfahren zum Nachweis dieser spezifischen DNA-Fragmente sind kommerziell erhältlich. Als zusätzliches Verfahren steht der "TUNEL assay" zur Verfügung, der DNA-Einzelstrangbrüche auch in Gewebeschnitten nachweisen kann. Zytotoxizität wird vor allem über eine veränderte Zellpermeabilität nachgewiesen, die als Marker für den Vitalitätszustand von Zellen dient. Dazu werden entweder im Zellkulturüberstand Marker analysiert, die normalerweise intrazellulär zu finden sind. Alternativ kann auch die Aufnahmefähigkeit von Farbmarkern analysiert werden, die von intakten Zellen nicht aufgenommen werden. Die bekanntesten Beispiele für Farbmarker sind Trypanblau und Propidiumiodid, ein üblicher intrazellulärer Marker ist die Laktatdehydrogenase, die im Überstand enzymatisch nachgewiesen werden kann. Unterschiedliche Testsysteme stehen von verschiedenen kommerziellen Anbietern (z.B. Roche Diagnostics, Invitrogene) zur Verfügung.

### 3. Migrationstests

Die Fähigkeit von Zellen zur Migration wird in einem spezifischen Migrationstest vorzugsweise mit Hilfe einer Boyden-Kammer (Corning Costar) analysiert (Cinamon G., Alon R. J. Immunol. Methods. 2003 Feb; 273(1-2):53-62; Stockton et al. 2001. Mol. Biol. Cell. 12: 1937-56). Dazu werden Zellen auf einem Filter mit spezifischer Porengröße kultiviert. Zellen, die migrieren können, sind in der Lage, durch diesen Filter in ein weiteres darunter liegendes Kulturgefäß zu wandern. Eine anschließende mikroskopische Analyse erlaubt dann die Bestimmung eines möglicherweise veränderten Migrationsverhaltens, dass durch den "gain of function" bzw. "loss of function" des Zielmoleküls induziert wurde.

### b. Funktionsanalyse in Tiermodellen

Alternativ zu Zellkulturexperimenten bieten sich zur Analyse der Zielgenfunktion aufwendige in vivo-Experimente in Tiermodellen an. Diese Modelle haben im Vergleich zu den zellbasierenden Verfahren den Vorteil, dass sie Fehlentwicklungen bzw. Krankheiten nachweisen können, die erst im Kontext des gesamten Organismus nachweisbar sind. Eine Vielzahl von Modellen für humane Erkrankungen ist inzwischen verfügbar (Abate-Shen & Shen. 2002. Trends in Genetics S1-5; Matsusue et. al. 2003. J. Clin. Invest. 111:737-47). Verschiedene Tiermodelle wie zum Beispiel Hefe, Nematoden oder Zebrafische sind inzwischen intensiv charakterisiert worden. Bevorzugte Modelle sind aber im Vergleich zu anderen Spezies Tiermodelle wie zum Beispiel die Maus (Mus musculus), weil sie die biologischen Prozesse im humanen Kontext am besten abbilden können. Für Mäuse sind in den letzten Jahren sowohl transgene Verfahren etabliert worden, die neue Gene in das Mausgenom integrieren ("gain of function"; Jegstrup I. et al. 2003. Lab Anim. 2003 Jan.;37(1):1-9). Alternativ werden durch andere methodische Ansätze Gene im Mausgenom ausgeschaltet und so ein Funktionsverlust eines gewünschten Gens induziert (knockout-Modelle, "loss of function"; Zambrowicz BP & Sands AT. 2003. Nat. Rev. Drug Discov. 2003 Jan;2(1):38-51; Niwa H. 2001. Cell Struct. Funct. 2001 Jun;26(3): 137-48.); technische Details sind vielfältig publiziert).

Nach Generierung der Mausmodelle können Veränderungen, die durch das Transgen bzw. durch den Funktionsverlust eines Gens induziert wurden, im Kontext des Gesamtorganismus analysiert werden (Balling R, 2001. Ann. Rev. Genomics Hum. Genet. 2:463-92). So sind zum Beispiel Verhaltenstests genauso wie biochemische Untersuchungen etablierter Blutparameter möglich. Histologische Analysen, Immunhistochemie oder die Elektronenmikroskopie ermöglichen die Charakterisierung von Veränderungen auf zellulärer Ebene. Das spezifische Expressionsmuster eines Genes kann durch eine in situ-Hybridisierung nachgewiesen werden (Peters et. al. 2003. Hum. Mol. Genet 12: 2109-20).

### Beispiel 1: Identifizierung des hypothetischen Proteins FLJ31461 als diagnostisches und therapeutisches Krebs-Target

FLJ31461 (SEQ ID NO: 1) mit der Genbank-Accession-Nummer NM_152454 wurde mittels Genprädiktionsprogrammen als putatives funktionell bisher nicht charakterisiertes Gen auf Chromosom 15 (15q25.3) ermittelt. Aus der in der Genbank hinterlegten Sequenz würden sich zwei mögliche offene Leserahmen ergeben. Der erste Leserahmen kodiert für ein Protein mit einer Länge von 136 Aminosäuren. Das Genprodukt (SEQ ID NO: 2), das in der RefSeq Datenbank des NCBI unter der Nummer NP_689667 abgelegt ist, hat somit ein kalkuliertes Molekulargewicht von ca. 15 kDa. Der zweite Leserahmen kodiert ein Protein mit einer Länge von 100 Aminosäuren (Nukleotidsequenz: SEQ ID NO: 7; Aminosäuresequenz: SEQ ID NO: 8).

Bei Sequenzanalysen des von uns klonierten FLJ31461-Gens stellten wir im Vergleich zu den in Datenbanken abgelegten Sequenzen überraschenderweise im kodierenden Bereich die Insertion eines Nukleotids fest. Dies resultiert in einer Leserahmenverschiebung. Zwei vollkommen neue offene Leseraster, die von den bereits in Sequenzdatenbanken hinterlegten Sequenzen nicht ableitbar sind, stellen sich dar. Dabei kodiert das neue Leseraster SEQ ID NO: 9 ein neues, 96 Aminosäure langes hypothetisches Protein (SEQ ID NO: 10). SEQ ID NO: 11 kodiert ein 133 Aminosäuren langes hypothetisches Protein (SEQ ID NO: 12). Da wir davon ausgehen müssen, dass die ursprünglichen Datenbankeingaben falsch sind, haben wir weitere Untersuchungen auf die SEQ ID NO: 9, SEQ ID NO: 10, SEQ ID NO: 11 und SEQ ID NO: 12 fokussiert.

Erfindungsgemäß wurde nach der Etablierung von FLJ31461-spezifischen quantitativen RT-PCR (Primer mit den SEQ ID NO: 3, 4, 13, 14, 15, 16) die Menge der genspezifischen Transkripte in gesundem Gewebe und in Karzinomproben untersucht (Abb. 1). FLJ31461 lässt sich mit Ausnahme von Testis in keinem der von uns untersuchten Normalgewebe nachweisen (Abb. 1A). FLJ31461 ist demnach mit hoher Wahrscheinlichkeit ein streng keimzellspezifisches Genprodukt. Überraschenderweise fanden wir bei der Analyse von Tumoren, dass FLJ31461 in vielen Tumortypen angeschaltet ist, während es in den korrespondierenden Normalgeweben unter der Detektionsgrenze ist (Abb. 1 A-D). Dies betrifft nicht nur fast alle von uns untersuchten Brusttumoren (Abb. 1C), sowie eine Reihe von Lungentumoren und Karzinomen des Hals-Nasen-Rachenbereiches, sondern auch andere Neoplasien in unterschiedlicher Häufigkeit (Abb. 1D).

FL131461 ist somit ein hochsepezifischer molekularer Marker für Tumorgewebe, der sowohl diagnostisch als auch therapeutisch genutzt werden kann. Als typischer Vertreter der Klasse der sog. Cancer/Testis-Antigene, die aufgrund ihrer selektiven Gewebeverteilung als Markerstrukturen dienen, kann dieses Genprodukt z.B. eine gezielte Targetierung von Tumorzellen ohne Schädigung von Normalgeweben gewährleisten. Cancer/Testis-Gene werden als attraktive Zielstrukturen für targetierte Therapien angesehen und bereits für spezifische immuntherapeutische Ansätze bei Krebserkrankungen in Phase I/II Studien getestet (z.B. Scanlan MJ, Gure AO, Jungbluth AA, Old U, Chen YT. 2002. Immunol Rev. 2002 Oct; 188: 22-32).

Um diese Daten auf Proteinebene zu bestätigen, wurden spezifische Antikörper bzw. Immunseren durch Immunisierung von Tieren generiert. Über Analyse der Transmembrandomänen von SEQ ID NO: 10 und SEQ ID NO: 12 mit bioinformatischen Werkzeugen (TMHMM, TMPRED) wurde die Proteintopologie prädiziert. So werden z.B. für die SEQ ID NO: 10 zwei Transmembrandomänen vorausgesagt; N- und C-Terminus des Proteins sind extrazellulär.

Erfindungsgemäß wurden für die Immunisierung Peptidepitope ausgewählt, insbesondere extrazellulär gelegene, die spezifisch für beide Proteinvarianten sind. Unter anderem wurden zur Immunisierung folgende Peptide für die Herstellung von Antikörpern ausgewählt: SEQ ID NO: 5, 6, 17 und 18.

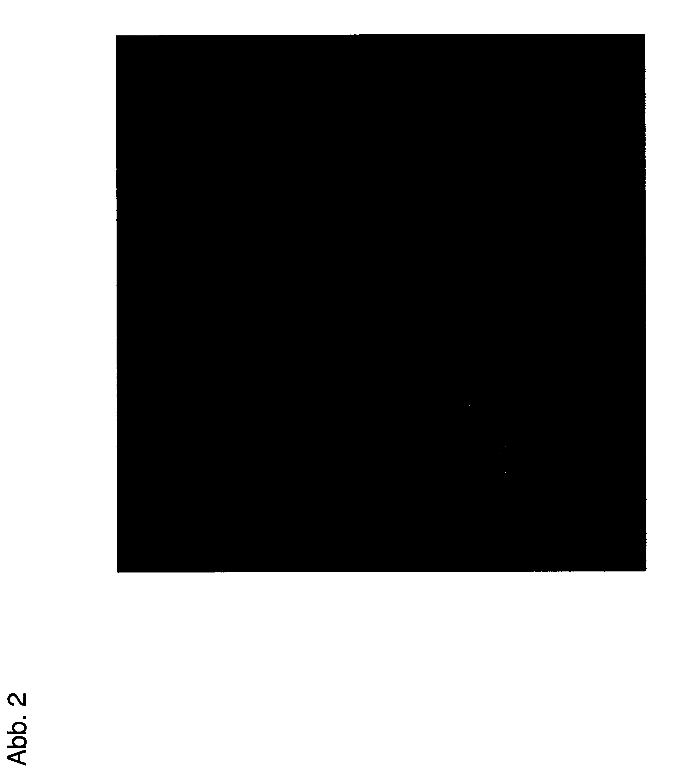

Beispielhaft werden die Daten für den Antikörper, der durch Immunisierung mit SEQ ID NO: 17 hergestellt wurde, dargestellt. Der spezifische Antikörper lässt sich unter verschiedenen Fixationsbedigungen für Immunfluoreszenz-Untersuchungen nutzen. Bei vergleichenden Färbungen von RT-PCR-positiven wie auch negativen Zelllinien ist das entsprechende Protein in gut nachweisbarer Menge spezifisch u.a. in den mittels quantitativer RT-PCR als positiv typisierten Brustkarzinomzellinien detektierbar (Abb. 2). Das endogene Protein stellt sich hier membranlokalisiert dar.

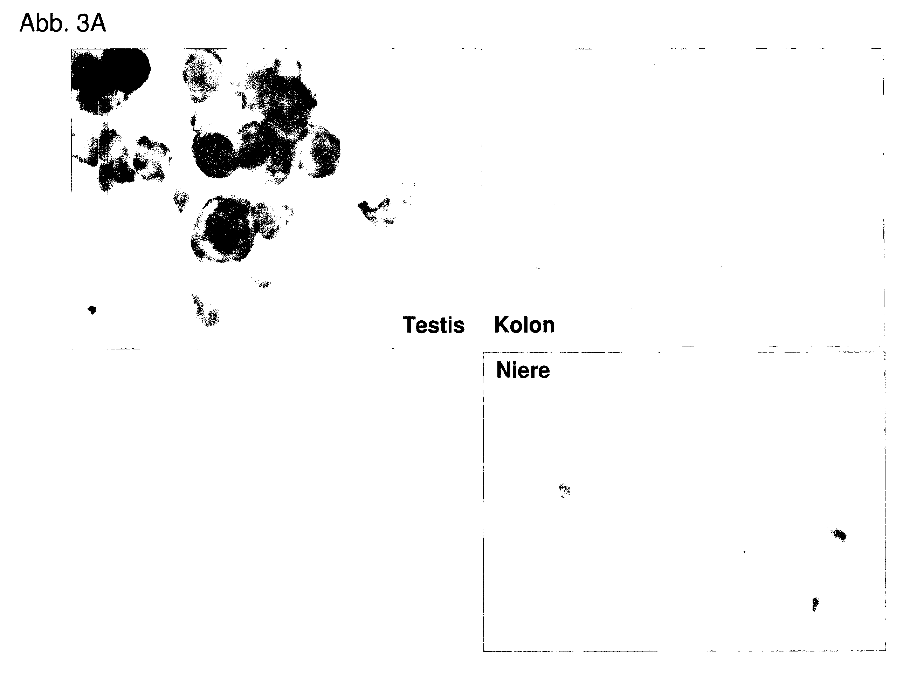

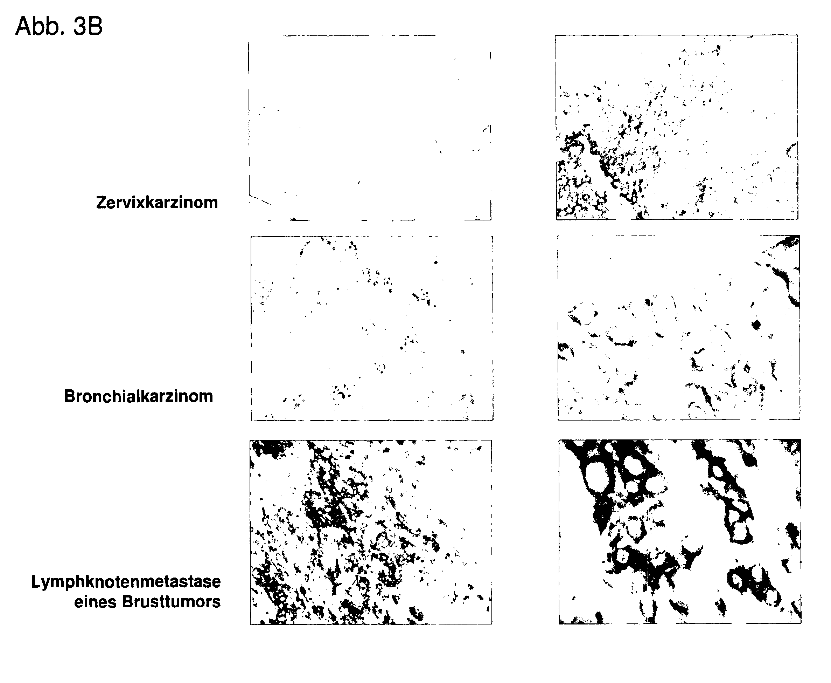

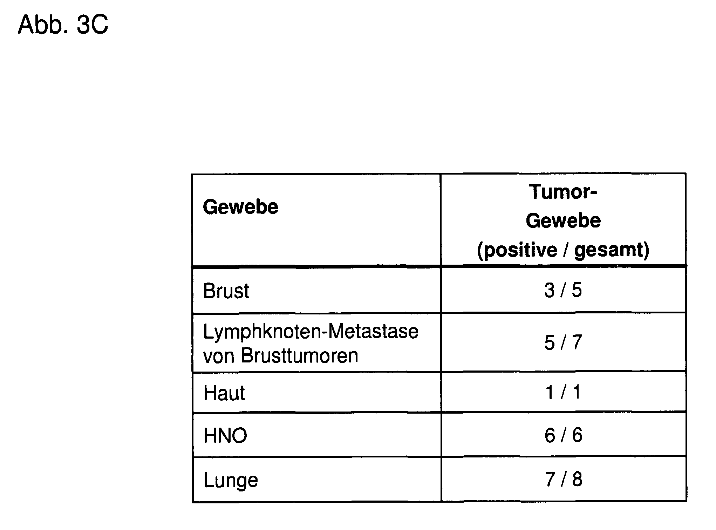

Solche Antikörper eigenen sich für die immunhistochemische Färbung von humanen Gewebeschnitten. Wir konnten in großen Teilen die auf Transkriptebene gefundene Gewebeverteilung bestätigen. Während wir außer in Testisgewebe kaum Reaktivität des Antikörpers in Normalgewebe feststellten (Abb. 3A), färben Antikörper gegen FLJ31461 verschiedenste menschliche Tumorpräparationen, unter diesen z.B. Brust- und Lungentumoren (Abb. 3B). Die Färbung der Zellen ist membranakzentuiert, was für die Lokalisierung des Proteins an der Zelloberfläche spricht. Überraschenderweise fanden wir, dass insbesondere Metastasen von Tumoren (Abb. 3B) besonders häufig und in einem hohen Anteil von Zellen dieses Protein exprimieren.

Diese Daten zeigen zum einen, dass dieses von uns gefundene Gen tatsächlich ein Protein bildet, dass dieses Protein hochspezifisch für humane Tumoren ist und sich auf der Oberflächenmembran solcher Tumorzellen befindet. Somit ist dieses Protein insbesondere für therapeutische Antikörper erreichbar. Unser Daten belegen ferner, dass spezifische Antikörper gegen dieses Protein hergestellt werden können. Diese Antikörper binden selektiv über den Marker FLJ31461 an Tumorzellen.

Erfindungsgemäß können derartige Antikörper für diagnostische Zwecke z.B. Immunhistologie genutzt werden. Insbesondere können solche Antikörper therapeutisch eingesetzt werden. Die hergestellten Antikörper können direkt auch zur Herstellung von chimären oder humanisierten rekombinanten Antikörpern verwendet werden. Dies kann auch direkt mit Antikörpern erfolgen, die aus Kaninchen gewonnen wurden (s. dazu J Biol Chem. 2000 May 5;275(18):13668-76 von Rader C, Ritter G, Nathan S, Elia M, Gout I, Jungbluth AA, Cohen LS, Welt S, Old LJ, Barbas CF 3rd. "The rabbit antibody repertoire as a novel source for the generation of therapeutic human antibodies"). Hierzu wurden von den immunisierten Tieren Lymphozyten asserviert. Auch für immuntherapeutische Verfahren wie Vakzinen bzw. den adoptiven Transfer von Antigen-spezifischen T-Lymphozyten stellt FLJ31461 ein hochattraktives Target dar.

## Patentansprüche

1. Pharmazeutische Zusammensetzung, umfassend einen oder mehrere Bestandteile, die aus der Gruppe ausgewählt sind, bestehend aus:
(i) einem Antikörper, der an ein Tumor-assoziiertes Antigen bindet,
(ii) einem Tumor-assoziierten Antigen oder einem Teil davon, der mindestens 6 aufeinanderfolgende Aminosäuren umfasst.
(iii) einer Nukleinsäure, die für ein Tumor-assozüertes Antigen oder einen Teil davon, der mindestens 6 aufeinanderfolgende Aminosäuren umfasst, kodiert,
(iv) einer Wirtszelle, die ein Tumor-assoziiertes Antigen oder einen Teil davon, der mindestens 6 aufeinanderfolgende Aminosäuren umfasst, exprimiert, und
(v) isolierten Komplexen zwischen einem Tumor-assoziierten Antigen oder einem Teil davon und einem HLA-Molekül,
wobei das Tumor-assoziierte Antigen
(a) die Aminosäuresequenz gemäß SEQ ID NO: 10 umfasst, oder
(b) eine Sequenz aufweist, die von einer Nukleinsäure kodiert wird, die mindestens 90% Identität zu der Nukleinsäure gemäß SEQ ID NO: 9 aufweist,
zur Behandlung von und/oder Vakzinierung gegen Krebs, der sich durch die Expression oder abnormale Expression des Tumor-assoziierten Antigens auszeichnet, wobei der Krebs ausgewählt ist aus der Gruppe bestehend aus Brusttumor, Lungentumor, Ösophagustumor, Kolontumor, Magentumor, Pankreastumor, Lebertumor, Ovarialtumor, Prostatatumor. HNO-Tumor, Nierentumor, Zervixtumor, Hauttumor und Uterustumor.

2. Pharmazeutische Zusammensetzung nach Anspruch 1, wobei der Antikörper selektiv an das Tumor-assoziierte Antigen bindet, tumorhemmende Aktivität aufweist und selektiv ist für Zellen, die eine Expression oder abnormale Expression des Tumor-assoziierten Antigens aufweisen.

3. Pharmazeutische Zusammensetzung nach Anspruch 1, wobei die Bestandteile unter (ii), (iii), (iv) und (v) selektiv die Menge an Komplexen zwischen einem HLA-Molekül und dem Tumor-assoziierten Antigen oder einem Teil davon erhöhen.

4. Pharmazeutische Zusammensetzung nach Anspruch 1 oder 3, wobei die Nukleinsäure unter (iii) in einem Expressionsvektor vorliegt.

5. Pharmazeutische Zusammensetzung nach Anspruch 1 oder 3, wobei die Wirtszelle das Tumor-assoziierte Antigen oder den Teil davon sekretiert.

6. Pharmazeutische Zusammensetzung nach Anspruch 1 oder 3, wobei die Wirtszelle zusätzlich ein HLA-Molekül exprimiert, das an das Tumor-assoziierte Antigen oder den Teil davon bindet.

7. Pharmazeutische Zusammensetzung nach Anspruch 6, wobei die Wirtszelle das HLA-Molekül und/oder das Tumor-assoziierte Antigen oder den Teil davon rekombinant exprimiert.

8. Pharmazeutische Zusammensetzung nach Anspruch 6, wobei die Wirtszelle das HLA-Molekül endogen exprimiert.

9. Pharmazeutische Zusammensetzung nach Anspruch 1, 3, 6 oder 8, wobei die Wirtszelle eine Antigen-präsentierende Zelle ist.

10. Pharmazeutische Zusammensetzung nach Anspruch 1 oder 2, wobei der Antikörper ein monoklonaler, chimärer oder humanisierter Antikörper oder ein Fragment eines Antikörpers ist.

11. Pharmazeutische Zusammensetzung nach Anspruch 1 oder 2, wobei der Antikörper mit einem therapeutischen oder diagnostischen Mittel gekoppelt ist.

12. Verfahren zur Diagnose einer Krebserkrankung, die sich durch die Expression oder abnormale Expression eines Tumor-assoziierten Antigens auszeichnet, umfassend den Nachweis:
(i) einer Nukleinsäure, die für das Tumor-assoziierte Antigen kodiert, und/oder
(ii) des Tumor-assoziierten Antigens
in einer aus einem Patienten isolierten biologischen Probe,
wobei das Tumor-assoziierte Antigen
(a) die Aminosäuresequenz gemäß SEQ ID NO: 10 umfasst, oder
(b) eine Sequenz aufweist, die von einer Nukleinsäure kodiert wird, die mindestens 90% Identität zu der Nukleinsäure gemäß SEQ ID NO: 9 aufweist,
wobei die Krebserkrankung ausgewählt ist aus der Gruppe bestehend aus Brusttumor, Lungentumor, Ösophagustumor, Kolontumor, Magentumor, Pankreastumor, Lebertumor, Ovarialtumor, Prostatatumor, HNO-Tumor, Nierentumor, Zervixtumor, Hauttumor und Uterustumor.

13. Verfahren zur Bestimmung der Regression, des Verlaufs oder des Ausbruchs einer Krebserkrankung, die sich durch die Expression oder abnormale Expression eines Tumor-assoziierten Antigens auszeichnet, umfassend die Überwachung einer Probe aus einem Patienten, der die Krebserkrankung aufweist oder in Verdacht steht, an der Krebserkrankung zu erkranken, in Bezug auf einen oder mehrere Parameter, ausgewählt aus der Gruppe, bestehend aus:
(i) der Menge der Nukleinsäure, die für das Tumor-assoziierte Antigen kodiert, und
(ii) der Menge des Tumor-assoziierten Antigens,
wobei das Tumor-assoziierte Antigen
(a) die Aminosäuresequenz gemäß SEQ ID NO: 10 umfasst, oder
(b) eine Sequenz aufweist, die von einer Nukleinsäure kodiert wird, die mindestens 90% Identität zu der Nukleinsäure gemäß SEQ ID NO: 9 aufweist,
wobei die Krebserkrankung ausgewählt ist aus der Gruppe bestehend aus Brusttumor, Lungentumor, Ösophagustumor, Kolontumor, Magentumor, Pankreastumor, Lebertumor, Ovarialtumor, Prostatatumor, HNO-Tumor, Nierentumor, Zervixtumor, Hauttumor und Uterustumor.

14. Verfahren nach Anspruch 13, wobei das Verfahren die Bestimmung des oder der Parameter zu einem ersten Zeitpunkt in einer ersten Probe und zu einem zweiten Zeitpunkt in einer weiteren Probe umfasst und durch einen Vergleich der beiden Proben der Verlauf der Krebserkrankung ermittelt wird.

15. Verfahren nach einem der Ansprüche 12-14, wobei der Nachweis oder die Überwachung gemäß (i) durch selektive Amplifikation der Nukleinsäure oder eines Teils davon oder mit einer Polynukleotid-Sonde erfolgt, die spezifisch mit der Nukleinsäure hybridisiert.

16. Verfahren nach einem der Ansprüche 12-14, wobei der Nachweis oder die Überwachung gemäß (ii) mit einem Antikörper erfolgt, der spezifisch an das Tumor-assoziierte Antigen bindet.

17. Verwendung eines Antikörpers, der an ein Tumor-assoziiertes Antigen bindet und mit einem therapeutischen oder diagnostischen Mittel gekoppelt ist, zur Herstellung einer pharmazeutischen Zusammensetzung zur Behandlung, Diagnose oder Überwachung einer Krebserkrankung, die sich durch die Expression oder abnormale Expression des Tumor-assoziierten Antigens auszeichnet,
wobei das Tumor-assoziierte Antigen
(a) die Aminosäuresequenz gemäß SEQ ID NO: 10 umfasst, oder
(b) eine Sequenz aufweist, die von einer Nukleinsäure kodiert wird, die mindestens 90% Identität zu der Nukleinsäure gemäß SEQ ID NO: 9 aufweist,
wobei die Krebserkrankung ausgewählt ist aus der Gruppe bestehend aus Brusttumor, Lungentumor, Ösophagustumor, Kolontumor, Magentumor, Pankreastumor, Leberturnor, Ovarialtumor, Prostatatumor, HNO-Tumor, Nierentumor, Zervixtumor, Hauttumor und Uterustumor.

18. Verfahren nach Anspruch 16 oder Verwendung nach Anspruch 17, wobei der Antikörper ein monoklonaler, chimärer oder humanisierter Antikörper oder ein Fragment eines Antikörpers ist.

## Claims

1. A pharmaceutical composition comprising one or more components selected from the group consisting of:
(i) an antibody which binds to a tumor-associated antigen,
(ii) a tumor-associated antigen or a fragment thereof which comprises at least 6 contiguous amino acids,
(iii) a nucleic acid encoding a tumor-associated antigen or a fragment thereof which comprises at least six contiguous amino acids,
(iv) a host cell expressing a tumor-associated antigen or a fragment thereof which comprises at least six contiguous amino acids and
(v) isolated complexes of a tumor-associated antigen or a fragment thereof and an HLA molecule,
wherein the tumor-associated antigen
(a) comprises the amino acid sequence according to SEQ ID NO: 10 or
(b) has a sequence which is encoded by a nucleic acid having at least 90% identity to the nucleic acid according to SEQ ID NO: 9
for the treatment of and/or vaccination against cancer **characterized by** the expression or abnormal expression of the tumor-associated antigen, wherein the cancer is selected from the group consisting of breast tumor, lung tumor, esophageal tumor, colon tumor, stomach tumor, pancreatic tumor, liver tumor, ovarian tumor, prostate tumor, ENT tumor, renal tumor, cervical tumor, skin tumor and uterine tumor.

2. The pharmaceutical composition according to claim 1, wherein the antibody binds selectively to the tumor-associated antigen, has tumor-inhibiting activity and is selective for cells expressing or abnormally expressing the tumor-associated antigen.

3. The pharmaceutical composition according to claim 1, wherein the components under (ii), (iii), (iv) and (v) selectively increase the amount of complexes of an HLA molecule and the tumor-associated antigen or a fragment thereof.

4. The pharmaceutical composition according to claim 1 or 3, wherein the nucleic acid under (iii) is present in an expression vector.

5. The pharmaceutical composition according to claim 1 or 3, wherein the host cell secretes the tumor-associated antigen or the fragment thereof.

6. The pharmaceutical composition according to claim 1 or 3, wherein the host cell additionally expresses an HLA molecule binding to the tumor-associated antigen or the fragment thereof.

7. The pharmaceutical composition according to claim 6, wherein the host cell expresses the HLA molecule and/or the tumor-associated antigen or the fragment thereof in a recombinant manner.

8. The pharmaceutical composition according to claim 6, wherein the host cell expresses the HLA molecule endogenously.

9. The pharmaceutical composition according to claim 1, 3, 6 or 8, wherein the host cell is an antigen-presenting cell.

10. The pharmaceutical composition according to claim 1 or 2, wherein the antibody is a monoclonal, chimeric or humanized antibody or a fragment of an antibody.

11. The pharmaceutical composition according to claim 1 or 2, wherein the antibody is coupled to a therapeutic or diagnostic agent.

12. A method for diagnosing a cancer disease **characterized by** the expression or abnormal expression of a tumor-associated antigen comprising the detection of:
(i) a nucleic acid encoding the tumor-associated antigen
and/or
(ii) the tumor-associated antigen
in a biological sample isolated from a patient,
wherein the tumor-associated antigen
(a) comprises the amino acid sequence according to SEQ ID NO: 10 or
(b) has a sequence which is encoded by a nucleic acid having at least 90% identity to the nucleic acid according to SEQ ID NO: 9,
wherein the cancer disease is selected from the group consisting of breast tumor, lung tumor, esophageal tumor, colon tumor, stomach tumor, pancreatic tumor, liver tumor, ovarian tumor, prostate tumor, ENT tumor, renal tumor, cervical tumor, skin tumor and uterine tumor.

13. A method for determining the regression, course or onset of a cancer disease **characterized by** the expression or abnormal expression of a tumor-associated antigen, said method comprising the monitoring of a sample from a patient having the cancer disease or being suspected of developing the cancer disease with respect to one or more parameters selected from the group consisting of:
(i) the amount of the nucleic acid encoding the tumor-associated antigen and
(ii) the amount of the tumor-associated antigen,
wherein the tumor-associated antigen
(a) comprises the amino acid sequence according to SEQ ID NO: 10 or
(b) has a sequence which is encoded by a nucleic acid having at least 90% identity to the nucleic acid according to SEQ ID NO: 9,
wherein the cancer disease is selected from the group consisting of breast tumor, lung tumor, esophageal tumor, colon tumor, stomach tumor, pancreatic tumor, liver tumor, ovarian tumor, prostate tumor, ENT tumor, renal tumor, cervical tumor, skin tumor and uterine tumor.

14. The method according to claim 13, wherein the method comprises the determination of the parameter or of the parameters at a first point in time in a first sample and at a second point in time in a further sample, wherein the course of the cancer disease is determined by comparing both samples.

15. The method according to any one of claims 12-14, wherein the detection or the monitoring according to (i) is effected by selectively amplifying the nucleic acid or a fragment thereof or by using a polynucleotide probe specifically hybridizing with the nucleic acid.

16. The method according to any one of claims 12-14, wherein the detection or the monitoring according to (ii) is effected by using an antibody binding specifically to the tumor-associated antigen.

17. A use of an antibody binding to a tumor-associated antigen and being coupled to a therapeutic or diagnostic agent for preparing a pharmaceutical composition for treating, diagnosing or monitoring a cancer disease **characterized by** the expression or abnormal expression of the tumor-associated antigen, wherein the tumor-associated antigen
(a) comprises the amino acid sequence according to SEQ ID NO: 10 or
(b) has a sequence which is encoded by a nucleic acid having at least 90% identity to the nucleic acid according to SEQ ID NO: 9,
wherein the cancer disease is selected from the group consisting of breast tumor, lung tumor, esophageal tumor, colon tumor, stomach tumor, pancreatic tumor, liver tumor, ovarian tumor, prostate tumor, ENT tumor, renal tumor, cervical tumor, skin tumor and uterine tumor.

18. The method according to claim 16 or the use according to claim 17, wherein the antibody is a monoclonal, chimeric or humanized antibody or a fragment of an antibody.

## Revendications

1. Composition pharmaceutique comprenant un ou plusieurs constituants, qui sont choisis dans le groupe consistant en:
(i) un anticorps qui se lie à un antigène associé à une tumeur,
(ii) un antigène associé à une tumeur ou une fraction de celui-ci, qui comprend au moins 6 acides aminés consécutifs,
(iii) un acide nucléique qui code pour un antigène associé à une tumeur ou une fraction de celui-ci, qui comprend au moins 6 acides aminés consécutifs,
(iv) une cellule hôte, qui exprime un antigène associé à une tumeur ou une fraction de celui-ci, qui comporte au moins 6 acides aminés consécutifs, et
(v) des complexes isolés entre un antigène associé à une tumeur ou une fraction de celui-ci et une molécule de HLA,
tandis que l'antigène associé à une tumeur
(a) englobe la séquence d'acides aminés selon SEQ ID NO: 10, ou
(b) présente une séquence qui est codée par un acide nucléique qui présente au moins 90% d'identité vis-à-vis de l'acide nucléique selon SEQ ID NO: 9,
pour le traitement d'un cancer et/ou la vaccination contre un cancer qui se **caractérise par** l'expression ou l'expression anormale de l'antigène associé à une tumeur, tandis que le cancer est choisi dans le groupe consistant en tumeur mammaire, tumeur pulmonaire, tumeur oesophagienne, tumeur du côlon, tumeur de l'estomac, tumeur du pancréas, tumeur hépatique, tumeur ovarienne, tumeur de la prostate, tumeur de HNO, tumeur rénale, tumeur du col utérin, tumeur de la peau et tumeur de l'utérus.

2. Composition pharmaceutique selon la revendication 1, dans laquelle l'anticorps se lie sélectivement à l'antigène associé à une tumeur, présente une activité d'inhibition des tumeurs et est sélectif pour les cellules qui présentent une expression ou une expression anormale de l'antigène associé à une tumeur.

3. Composition pharmaceutique selon la revendication 1, dans laquelle les constituants indiqués sous (ii), (iii), (iv) et (v) augmentent sélectivement la quantité de complexes entre une molécule de HLA et l'antigène associé à une tumeur ou une fraction de celui-ci.

4. Composition pharmaceutique selon la revendication 1 ou 3, dans laquelle l'acide nucléique sous (iii) est présent dans un vecteur d'expression.

5. Composition pharmaceutique selon la revendication 1 ou 3, dans laquelle la cellule hôte sécrète l'antigène associé à une tumeur ou la fraction de celui-ci.

6. Composition pharmaceutique selon la revendication 1 ou 3, dans laquelle la cellule hôte exprime en outre une molécule de HLA qui se lie à l'antigène associé à une tumeur ou à la fraction de celui-ci.

7. Composition pharmaceutique selon la revendication 6, dans laquelle la cellule hôte exprime la molécule de HLA et/ou l'antigène associé à une tumeur ou la fraction de celui-ci de manière recombinante.

8. Composition pharmaceutique selon la revendication 6, dans laquelle la cellule hôte exprime la molécule de HLA de manière endogène.

9. Composition pharmaceutique selon la revendication 1, 3, 6 ou 8, dans laquelle la cellule hôte est une cellule présentant l'antigène.

10. Composition pharmaceutique selon la revendication 1 ou 2, dans laquelle l'anticorps est un anticorps monoclonal ou un fragment d'un anticorps, monoclonal, chimère ou humanisé.

11. Composition pharmaceutique selon la revendication 1 ou 2, dans laquelle l'anticorps est couplé avec un agent thérapeutique ou diagnostique.

12. Procédé pour le diagnostic d'une affection cancéreuse, qui se **caractérise par** l'expression ou l'expression anormale d'un antigène associé à une tumeur, comprenant la détermination de:
(i) un acide nucléique qui code pour l'antigène associé à une tumeur, et/ou
(ii) l'antigène associé à une tumeur,
dans un échantillon biologique isolé à partir d'un patient,
tandis que l'antigène associé à une tumeur
(a) englobe la séquence d'acides aminés selon SEQ ID NO: 10, ou
(b) présente une séquence qui est codée par un acide nucléique qui présente au moins 90% d'identité vis-à-vis de l'acide nucléique selon SEQ ID NO: 9,
tandis que l'affection cancéreuse est choisie dans le groupe consistant en tumeur mammaire, tumeur pulmonaire, tumeur oesophagienne, tumeur du côlon, tumeur de l'estomac, tumeur du pancréas, tumeur hépatique, tumeur ovarienne, tumeur de la prostate, tumeur de HNO, tumeur rénale, tumeur du col utérin, tumeur de la peau et tumeur de l'utérus.

13. Procédé pour la détermination de la régression, de l'évolution ou de l'apparition d'une affection cancéreuse, qui se **caractérise par** l'expression ou l'expression anormale d'un antigène associé à une tumeur, comprenant le contrôle d'un échantillon provenant d'un patient, qui présente l'affection cancéreuse ou qui est soupçonné d'être atteint par l'affection cancéreuse, relativement à un ou plusieurs paramètres, choisis dans le groupe consistant en
(i) la quantité d'acide nucléique qui code pour l'antigène associé à une tumeur, et
(ii) la quantité de l'antigène associé à une tumeur,
tandis que l'antigène associé à une tumeur
(a) englobe la séquence d'acides aminés selon SEQ ID NO:10, ou
(b) présente une séquence qui est codée par un acide nucléique qui présente au moins 90% d'identité vis-à-vis de l'acide nucléique selon SEQ ID NO: 9,
tandis que l'affection cancéreuse est choisie dans le groupe consistant en tumeur mammaire, tumeur pulmonaire, tumeur oesophagienne, tumeur du côlon, tumeur de l'estomac, tumeur du pancréas, tumeur hépatique, tumeur ovarienne, tumeur de la prostate, tumeur de HNO, tumeur rénale, tumeur du col utérin, tumeur de la peau et tumeur de l'utérus.

14. Procédé selon la revendication 13, dans lequel le procédé comprend la détermination du ou des paramètres à un premier instant dans un premier échantillon et à un deuxième instant dans un autre échantillon, et on détermine le déroulement de l'affection cancéreuse par comparaison des deux échantillons.

15. Procédé selon l'une des revendications 12-14, dans lequel la détermination ou la surveillance selon (i) s'effectue par amplification sélective de l'acide nucléique ou d'une fraction de celui-ci ou avec une sonde polynucléotidique qui hybride spécifiquement avec l'acide nucléique.

16. Procédé selon l'une des revendications 12-14, dans lequel la détermination ou la surveillance selon (ii) s'effectue avec un anticorps qui se lie spécifiquement à l'antigène associé à une tumeur.

17. Utilisation d'un anticorps qui se lie à un antigène associé à une tumeur et est couplé avec un agent thérapeutique ou diagnostique, pour la préparation d'une composition pharmaceutique pour le traitement, le diagnostic ou la surveillance d'une affection cancéreuse, qui se **caractérise par** l'expression ou l'expression anormale de l'antigène associé à une tumeur,
tandis que l'antigène associé à une tumeur
(a) englobe la séquence d'acides aminés selon SEQ ID NO: 10, ou
(b) présente une séquence qui est codée par un acide nucléique qui présente au moins 90% d'identité vis-à-vis de l'acide nucléique selon SEQ ID NO: 9,
tandis que l'affection cancéreuse est choisie dans le groupe consistant en tumeur mammaire, tumeur pulmonaire, tumeur oesophagienne, tumeur du côlon, tumeur de l'estomac, tumeur du pancréas, tumeur hépatique, tumeur ovarienne, tumeur de la prostate, tumeur de HNO, tumeur rénale, tumeur du col utérin, tumeur de la peau et tumeur de l'utérus.

18. Procédé selon la revendication 16 ou utilisation selon la revendication 17, dans lequel l'anticorps est un anticorps ou un fragment d'un anticorps, monoclonal, chimère ou humanisé.


### SEQUENZPROTOKOLL

<110> Ganymed Pharmaceuticals AG
<120> Identifizierung von oberflächen-assoziierten Antigenen für die Tumordiagnose und -therapie
<130> 342-20 PCT EP
<150> DE 10 2004 023 187.7
   <151> 2004-05-11
<160> 18
<170> PatentIn version 3.3
<210> 1
   <211> 1785
   <212> DNA
   <213> Homo sapiens
<220>
   <221> CDS
   <222> (204)..(614)
<400> 1
   actcagctct ctcaccatgc gattgccctg caacaccttg gaactctgca gagagtcccc     60
   agcaggaagg tctcacctga ggtgaacctt cgaccttgga cttctcagcc tccagaatga    120
   agaatggcaa ccatcaaatc aagaaattgg cccaaagccc tacagtctgc aaacatcata    180
   acaattcatc ctgaagtttc tcc atg aat tgt gat gct ttg cta cat cat tct    233
                             Met Asn Cys Asp Ala Leu Leu His His Ser
                             1               5                   10
   gca atc cca gaa gat ttt ttg cat att ttt ttg cta tta cag aaa atc      281
   Ala Ile Pro Glu Asp Phe Leu His Ile Phe Leu Leu Leu Gln Lys Ile
                   15                  20                  25
   tca gtc tcc ctc cct ctc tct ctc tct caa tct gtg tgt ctc ttt tac      329
   Ser Val Ser Leu Pro Leu Ser Leu Ser Gln Ser Val Cys Leu Phe Tyr
               30                  35                  40
   tcc ata tct ctg tgt gtg tct ctt tta ctc cat atc tct ctg tgt gtg      377
   Ser Ile Ser Leu Cys Val Ser Leu Leu Leu His Ile Ser Leu Cys Val
           45                  50                  55
   tct gtt tat gtc tct ctc tct ctc tca tcc ttc cca tgt ttc tct ctc      425
   Ser Val Tyr Val Ser Leu Ser Leu Ser Ser Phe Pro Cys Phe Ser Leu
       60                  65                  70
   aca cac aca cac act cat tca cag ctt tca aaa gac acg tct gtc ctt       473
   Thr His Thr His Thr His Ser Gln Leu Ser Lys Asp Thr Ser Val Leu
   75                  80                  85                  90
   acc ttc act ttt tgt ttt aaa cag cac act cac ttt act ctg aac tat      521
   Thr Phe Thr Phe Cys Phe Lys Gln His Thr His Phe Thr Leu Asn Tyr
                   95                  100                 105
   acc tca cat gca cac gag ctt tct gct cca tct gtt cat ccc aca tgt      569
   Thr Ser His Ala His Glu Leu Ser Ala Pro Ser Val His Pro Thr Cys
               110                 115                 120
   gtc ttc aca ttc aaa gca gca cct tcc cca aga cca gct acc taa          614
   Val Phe Thr Phe Lys Ala Ala Pro Ser Pro Arg Pro Ala Thr
           125                 130                 135
   ccacctccca cctccacccc atccctagtc agaggaaggc ctggttccca cctgaattca    674
   gctttgtcaa agagcctcct ggaaagctgt catcttcagt tagtagggat aatgggatta    734
   ttctatctgt gtaataataa catgttcaat ttaaagaaaa aaatctgaag ccacttaaaa    794
   gctactgttt ggcaccgata cattattcca gtaatgaata atcattaaag atattattct    854
   ggatgcagtt accatgcagt gatgtgaata aaatgcatta gatggaaaat tgtatttcaa    914
   gtaaatatat gcactggtag aaatgtatta ccacccacta atatgtatta attcaaaacc    974
   aaatgccaac tggagttcgc ctacacgggt ttgaatggca ggcagtgatt tggaagtggg   1034
   aggaaatagg tttggatttg gtcaaataga ctgagaagtg atagtggggg cgggggttta   1094
   tgactcaaac tttaacaggt gagaagacta tgccatggac agaacaggca tgaggggctc   1154
   ccctcctacg cctctttaag agatttttat ctctgactaa ggattactgg tagttgttga   1214
   catttctgaa gcagtggatc tttttccttt ttcactatct gcatcttcaa atattctttt   1274
   ctgaagaaag ttaaaaggaa gcctgtacat tttttgctaa ggtaaatgcc ttgccatctt   1334
   atttcatttt ctcatttttt tcttcagtgc acaacataag caactgtcct ccttgtcata   1394
   ctcaagatga gcttggcata tctgaaatct gcagggattt tctcattagc acagggttcc   1454
   aagcccaaac cgtgaagatg gagttttcat ttttaaatgg cacatcttca agttcttgcc   1514
   ctgtcctcac tttagtatgc cccagaggaa gtcaaagata tggacactct aagactcaga   1574
   agaactttct caggcattca ttttcctatc tattttgagc cattttattt aaaaggttac   1634
   aattttaaac ctctctttaa ttaaaagata ccagagttac aatgcaatac tatttggcaa   1694
   tcaaaactaa tgaagcacag atgcatgcta caacacgaat gaactttgaa acgttgtgtt   1754
   aagtgaaata aaccagttat tatacaaggc c                                  1785
<210> 2
   <211> 136
   <212> PRT
   <213> Homo sapiens
<400> 2
   Met Asn Cys Asp Ala Leu Leu His His Ser Ala Ile Pro Glu Asp Phe
   1               5                   10                  15
   Leu His Ile Phe Leu Leu Leu Gln Lys Ile Ser Val Ser Leu Pro Leu
               20                  25                  30
   Ser Leu Ser Gln Ser Val Cys Leu Phe Tyr Ser Ile Ser Leu Cys Val
           35                  40                  45
   Ser Leu Leu Leu His Ile Ser Leu Cys Val Ser Val Tyr Val Ser Leu
       50                  55                  60
   Ser Leu Ser Ser Phe Pro Cys Phe Ser Leu Thr His Thr His Thr His
   65                  70                  75                  80
   Ser Gln Leu Ser Lys Asp Thr Ser Val Leu Thr Phe Thr Phe Cys Phe
   85 90 95
   Lys Gln His Thr His Phe Thr Leu Asn Tyr Thr Ser His Ala His Glu
   100 105 110
   Leu Ser Ala Pro Ser Val His Pro Thr Cys Val Phe Thr Phe Lys Ala
   115 120 125
   Ala Pro Ser Pro Arg Pro Ala Thr
   130 135
<210> 3
   <211> 21
   <212> DNA
   <213> künstliche Sequenz
<220>
   <223> Beschreibung der künstlichen Sequenz: oligonukleotid
<400> 3
   cagcctccag aatgaagaat g 21
<210> 4
   <211> 18
   <212> DNA
   <213> künstliche Sequenz
<220>
   <223> Beschreibung der künstlichen Sequenz: oligonukleotid
<400> 4
   ggagggagac tgagattt 18
<210> 5
   <211> 15
   <212> PRT
   <213> Homo sapiens
<400> 5
   Lys Ile Ser Val Ser Leu Pro Leu Ser Leu Ser Gln Ser Val Cys
   1 5 10 15
<210> 6
   <211> 14
   <212> PRT
   <213> Homo sapiens
<400> 6
   Gln Leu Ser Lys Asp Thr Ser Val Leu Thr Phe Thr Phe Cys
   1 5 10
<210> 7
   <211> 303
   <212> DNA
   -<213> Homo sapiens
<220>
   <221> CDS
   <222> (1)..(303)
<400> 7
   atg ctt tgc tac atc att ctg caa tcc cag aag att ttt tgc ata ttt          48
   Met Leu Cys Tyr Ile Ile Leu Gln Ser Gln Lys Ile Phe Cys Ile Phe
   1               5                   10                  15
   ttt tgc tat tac aga aaa tct cag tct ccc tcc ctc tct ctc tct ctc          96
   Phe Cys Tyr Tyr Arg Lys Ser Gln Ser Pro Ser Leu Ser Leu Ser Leu
               20                  25                  30
   aat ctg tgt gtc tct ttt act cca tat ctc tgt gtg tgt ctc ttt tac         144
   Asn Leu Cys Val Ser Phe Thr Pro Tyr Leu Cys Val Cys Leu Phe Tyr
           35                  40                  45
   tcc ata tct ctc tgt gtg tgt ctg ttt atg tct ctc tct ctc tct cat         192
   Ser Ile Ser Leu Cys Val Cys Leu Phe Met Ser Leu Ser Leu Ser His
       50                  55                  60
   cct tcc cat gtt tct ctc tca cac aca cac aca ctc att cac agc ttt         240
   Pro Ser His Val Ser Leu Ser His Thr His Thr Leu Ile His Ser Phe
   65                  70                  75                  80
   caa aag aca cgt ctg tcc tta cct tca ctt ttt gtt tta aac agc aca         288
   Gln Lys Thr Arg Leu Ser Leu Pro Ser Leu Phe Val Leu Asn Ser Thr
                   85                  90                  95
   ctc act tta ctc tga                                                     303
   Leu Thr Leu Leu
               100
<210> 8
   <211> 100
   <212> PRT
   <213> Homo sapiens
<400> 8
   Met Leu Cys Tyr Ile Ile Leu Gln Ser Gln Lys Ile Phe Cys Ile Phe
   1               5                   10                  15
   Phe Cys Tyr Tyr Arg Lys Ser Gln Ser Pro Ser Leu Ser Leu Ser Leu
               20                  25                  30
   Asn Leu Cys Val Ser Phe Thr Pro Tyr Leu Cys Val Cys Leu Phe Tyr
           35                  40                  45
   Ser Ile Ser Leu Cys Val Cys Leu Phe Met Ser Leu Ser Leu Ser His
       50                  55                  60
   Pro Ser His Val Ser Leu Ser His Thr His Thr Leu Ile His Ser Phe
   65                  70                  75                  80
   Gln Lys Thr Arg Leu Ser Leu Pro Ser Leu Phe Val Leu Asn Ser Thr
                   85                  90                  95
   Leu Thr Leu Leu
               100
<210> 9
   <211> 1786
   <212> DNA
   <213> Homo sapiens
<220>
   <221> CDS
   <222> (204)..(494)
<400> 9
   actcagctct ctcaccatgc gattgccctg caacaccttg gaactctgca gagagtcccc      60
   agcaggaagg tctcacctga ggtgaacctt cgaccttgga cttctcagcc tccagaatga     120
   agaatggcaa ccatcaaatc aagaaattgg cccaaagccc tacagtctgc aaacatcata     180
   acaattcatc ctgaagtttc tcc atg aat tgt gat gct ttg cta cat cat tct     233
                             Met Asn Cys Asp Ala Leu Leu His His Ser
                             1               5                   10
   gca atc cca gaa gat ttt ttg cat att ttt ttg cta tta cag aaa aat      281
   Ala Ile Pro Glu Asp Phe Leu His Ile Phe Leu Leu Leu Gln Lys Asn
                   15                  20                  25
   ctc agt ctc cct ccc tct ctc tct ctc tca atc tgt gtg tct ctt tta      329
   Leu Ser Leu Pro Pro Ser Leu Ser Leu Ser Ile Cys Val Ser Leu Leu
               30                  35                  40
   ctc cat atc tct gtg tgt gtc tct ttt act cca tat ctc tct gtg tgt      377
   Leu His Ile Ser Val Cys Val Ser Phe Thr Pro Tyr Leu Ser Val Cys
           45                  50                  55
   gtc tgt tta tgt ctc tct ctc tct ctc atc ctt ccc atg ttt ctc tct      425
   Val Cys Leu Cys Leu Ser Leu Ser Leu Ile Leu Pro Met Phe Leu Ser
       60                  65                  70
   cac aca cac aca cac tca ttc aca gct ttc aaa aga cac gtc tgt cct      473
   His Thr His Thr His Ser Phe Thr Ala Phe Lys Arg His Val Cys Pro
   75                  80                  85                  90
   tac ctt cac ttt ttg ttt taa acagcacact cactttactc tgaactatac         524
   Tyr Leu His Phe Leu Phe
                   95
   ctcacatgca cacgagcttt ctgctccatc tgttcatccc acatgtgtct tcacattcaa    584
   agcagcacct tccccaagac cagctaccta accacctccc acctccaccc catccctagt    644
   cagaggaagg cctggttccc acctgaattc agctttgtca aagagcctcc tggaaagctg    704
   tcatcttcag ttagtaggga taatgggatt attctatctg tgtaataata acatgttcaa    764
   tttaaagaaa aaaatctgaa gccacttaaa agctactgtt tggcaccgat acattattcc    824
   agtaatgaat aatcattaaa gatattattc tggatgcagt taccatgcag tgatgtgaat    884
   aaaatgcatt agatggaaaa ttgtatttca agtaaatata tgcactggta gaaatgtatt    944
   accacccact aatatgtatt aattcaaaac caaatgccaa ctggagttcg cctacacggg   1004
   tttgaatggc aggcagtgat ttggaagtgg gaggaaatag gtttggattt ggtcaaatag   1064
   actgagaagt gatagtgggg gcgggggttt atgactcaaa ctttaacagg tgagaagact   1124
   atgccatgga cagaacaggc atgaggggct cccctcctac gcctctttaa gagattttta   1184
   tctctgacta aggattactg gtagttgttg acatttctga agcagtggat ctttttcctt   1244
   tttcactatc tgcatcttca aatattcttt tctgaagaaa gttaaaagga agcctgtaca   1304
   ttttttgcta aggtaaatgc cttgccatct tatttcattt tctcattttt ttcttcagtg   1364
   cacaacataa gcaactgtcc tccttgtcat actcaagatg agcttggcat atctgaaatc   1424
   tgcagggatt ttctcattag cacagggttc caagcccaaa ccgtgaagat ggagttttca   1484
   tttttaaatg gcacatcttc aagttcttgc cctgtcctca ctttagtatg ccccagagga   1544
   agtcaaagat atggacactc taagactcag aagaactttc tcaggcattc attttcctat   1604
   ctattttgag ccattttatt taaaaggtta caattttaaa cctctcttta attaaaagat   1664
   accagagtta caatgcaata ctatttggca atcaaaacta atgaagcaca gatgcatgct   1724
   acaacacgaa tgaactttga aacgttgtgt taagtgaaat aaaccagtta ttatacaagg   1784
   cc                                                                  1786
<210> 10
   <211> 96
   <212> PRT
   <213> Homo sapiens
<400> 10
   Met Asn Cys Asp Ala Leu Leu His His Ser Ala Ile Pro Glu Asp Phe
   1               5                   10                  15
   Leu His Ile Phe Leu Leu Leu Gln Lys Asn Leu Ser Leu Pro Pro Ser
               20                  25                  30
   Leu Ser Leu Ser Ile Cys Val Ser Leu Leu Leu His Ile Ser Val Cys
           35                  40                  45
   Val Ser Phe Thr Pro Tyr Leu Ser Val Cys Val Cys Leu Cys Leu Ser
       50                  55                  60
   Leu Ser Leu Ile Leu Pro Met Phe Leu Ser His Thr His Thr His Ser
   65                  70                  75                  80
   Phe Thr Ala Phe Lys Arg His Val Cys Pro Tyr Leu His Phe Leu Phe
                   85                  90                  95
<210> 11
   <211> 402
   <212> DNA
   <213> Homo sapiens
<220>
   <221> CDS
   <222> (1)..(402)
<400> 11
   atg ctt tgc tac atc att ctg caa tcc cag aag att ttt tgc ata ttt      48
   Met Leu Cys Tyr Ile Ile Leu Gln Ser Gln Lys Ile Phe Cys Ile Phe
   1               5                   10                  15
   ttt tgc tat tac aga aaa atc tca gtc tcc ctc cct ctc tct ctc tct      96
   Phe Cys Tyr Tyr Arg Lys Ile Ser Val Ser Leu Pro Leu Ser Leu Ser
               20                  25                  30
   caa tct gtg tgt ctc ttt tac tcc ata tct ctg tgt gtg tct ctt tta     144
   Gln Ser Val Cys Leu Phe Tyr Ser Ile Ser Leu Cys Val Ser Leu Leu
           35                  40                  45
   ctc cat atc tct ctg tgt gtg tct gtt tat gtc tct ctc tct ctc tca     192
   Leu His Ile Ser Leu Cys Val Ser Val Tyr Val Ser Leu Ser Leu Ser
       50                  55                  60
   tcc ttc cca tgt ttc tct ctc aca cac aca cac act cat tca cag ctt     240
   Ser Phe Pro Cys Phe Ser Leu Thr His Thr His Thr His Ser Gln Leu
   65                  70                  75                  80
   tca aaa gac acg tct gtc ctt acc ttc act ttt tgt ttt aaa cag cac     288
   Ser Lys Asp Thr Ser Val Leu Thr Phe Thr Phe Cys Phe Lys Gln His
                   85                  90                  95
   act cac ttt act ctg aac tat acc tca cat gca cac gag ctt tct gct      336
   Thr His Phe Thr Leu Asn Tyr Thr Ser His Ala His Glu Leu Ser Ala
               100                 105                 110
   cca tct gtt cat ccc aca tgt gtc ttc aca ttc aaa gca gca cct tcc      384
   Pro Ser Val His Pro Thr Cys Val Phe Thr Phe Lys Ala Ala Pro Ser
           115                 120                 125
   cca aga cca gct acc taa                                              402
   Pro Arg Pro Ala Thr
       130
<210> 12
   <211> 133
   <212> PRT
   <213> Homo sapiens
<400> 12
   Met Leu Cys Tyr Ile Ile Leu Gln Ser Gln Lys Ile Phe Cys Ile Phe
   1               5                   10                  15
   Phe Cys Tyr Tyr Arg Lys Ile Ser Val Ser Leu Pro Leu Ser Leu Ser
               20                  25                  30
   Gln Ser Val Cys Leu Phe Tyr Ser Ile Ser Leu Cys Val Ser Leu Leu
           35                  40                  45
   Leu His Ile Ser Leu Cys Val Ser Val Tyr Val Ser Leu Ser Leu Ser
       50                  55                  60
   Ser Phe Pro Cys Phe Ser Leu Thr His Thr His Thr His Ser Gln Leu
   65                  70                  75                  80
   Ser Lys Asp Thr Ser Val Leu Thr Phe Thr Phe Cys Phe Lys Gln His
                   85                  90                  95
   Thr His Phe Thr Leu Asn Tyr Thr Ser His Ala His Glu Leu Ser Ala
               100                 105                 110
   Pro Ser Val His Pro Thr Cys Val Phe Thr Phe Lys Ala Ala Pro Ser
           115                 120                 125
   Pro Arg Pro Ala Thr
       130
<210> 13
   <211> 26
   <212> DNA
   <213> künstliche Sequenz
<220>
   <223> Beschreibung der künstlichen Sequenz: oligonukleotid
<400> 13
   caccatgaat tgtgatgctt tgctac 26
<210> 14
   <211> 22
   <212> DNA
   <213> künstliche Sequenz
<220>
   <223> Beschreibung der künstlichen Sequenz: oligonukleotid
<400> 14
   ctaggtagct ggtcttgggg aa 22
<210> 15
   <211> 21
   <212> DNA
   <213> künstliche Sequenz
<220>
   <223> Beschreibung der künstlichen Sequenz: oligonukleotid
<400> 15
   ttctcagcct ccagaatgaa g 21
<210> 16
   <211> 21
   <212> DNA
   <213> künstliche Sequenz
<220>
   <223> Beschreibung der künstlichen sequenz: oligonukleotid
<400> 16
   gaaggtaagg acagacgtgt c 21
<210> 17
   <211> 12
   <212> PRT
   <213> Homo sapiens
<400> 17
   Thr His Ser Phe Thr Ala Phe Lys Arg His Val Cys
   1               5                   10
<210> 18
   <211> 13
   <212> PRT
   <213> Homo sapiens
<400> 18
   Asn Leu Ser Leu Pro Pro Ser Leu Ser Leu Ser Ile Cys
   1               5                   10